# EUROPEAN PATENT APPLICATION

(11) **EP 2 208 725 A1**
(43) Date of publication of application: **21.07.2010**
(21) Application number: 09305030.0
(22) Date of filing: 13.01.2009
(51) Int. Cl.: C07D 207/38, C07D 401/04, C07D 401/06, C07D 403/10, C07D 405/04, C07D 405/06, C07D 409/06, A61K 31/4015, A61P 35/00

(54) **Novel specific inhibitors of ubiquitin specific protease 7, the pharmaceutical compositions thereof and their therapeutic applications**

(71) Applicant: Hybrigenics S.A., 75014 Paris (FR)
(72) Inventor: Lopez, Roman, 92130 Issy-Les-Moulineaux (FR); Collura, Vincent, 92290 Chatenay Malabry (FR); Sippl, Wolfgang, 06120 Halle (DE); Colland, Frédéric, 95380 Puiseux en France (FR)
(74) Representative: Jacobson, Claude

(57) **Abstract**

The present invention concerns new compounds of formula (I), their process of preparation and their therapeutic use.

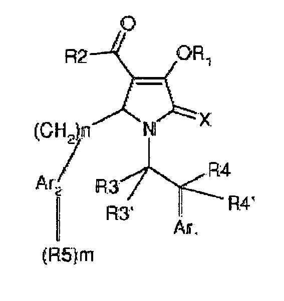

## Description

The present invention concerns new specific inhibitors of ubiquitin specific proteases, their process of preparation and their therapeutic use.

Ubiquitin specific proteases (USP) are cysteines proteases which belong to the de-Ubiquitinylation enzymes (DUBs) family.

Deregulation of the ubiquitin-proteasome system has been implicated in the pathogenesis of many human diseases, including cancer (Hoeller et al. Nat Rev Cancer 2006, 6(10), 776-788), neurodegenerative disorders (Rubinsztein, Nature 2006, 443(7113), 780-786) and viral diseases (Gao & Luo Can J Physiol Pharmacol 2006, 84(1), 5-14). The recent approval of the proteasome inhibitor Velcade^{®} (bortezomib) for the treatment of multiple myeloma and mantle cell lymphoma has established this system as a valid target for cancer treatment (Adams, Nat Rev Cancer 2004, 4(5), 349-360). A promising alternative to targeting the proteasome itself would be to interfere with the upstream ubiquitin conjugation/deconjugation machinery, to generate more specific, less toxic anticancer agents.

Mono- and polyubiquitination can be reversed by deubiquitinating enzymes, which specifically cleave the isopeptide bond at the C-terminus of ubiquitin. Ubiquitin specific proteases and ubiquitin C-terminal hydrolases (UCH) enzymes are the best characterized members of the DUB family (Nijman et al. Cell 2005, 123(5), 773-786). UCHs are thought to cleave small protein substrates preferentially and to be involved principally in the processing and recycling of ubiquitin, but their specific functions remain poorly understood. USPs constitute the largest subfamily of DUBs, with more than 60 members. They remove ubiquitin from specific protein substrates, thus preventing their targeting to the proteasome or regulating their subcellular localization and activation (Daviet & Colland, Biochimie 2008, 90(2), 270-83. USPs are emerging as potential targets for pharmacological interference with the ubiquitin regulation machinery, based on their protease activity and involvement in several human diseases.

USP7 (Ubiquitin Specific Protease 7)/HAUSP (Herpes Associated Ubiquitin Specific Protease) is a 135 kDa protein of the USP family. USP7 has been shown to interact with viral proteins, such as ICP0 (Vmw 110), a herpes simplex virus immediate-early gene stimulating initiation of the viral lytic cycle (Everett et al., J Virol 73, 1999, 417-426), and EBNA1 (Epstein-Barr Nuclear Antigen-1) (Holowaty et al., J Biol Chem 2003, 278, 29987-29994 and 47753-47761). Human proteins, such as p53 and the major E3 ligase of p53, Mdm2, have also been identified as partners and substrates of USP7 (Cummins et al. Nature 2004, 486, Cummins & Vogelstein, Cell Cycle, 2004, 3, 689-692; Li et al. Mol Cell 2004, 13, 879-886; Li et al. Nature 2002, 416, 648-653). More generally USP7 can deubiquitinate different targets, including Mdm2 and p53, and the net deubiquitination of these latter targets ultimately determines functional p53 levels. Consistent with recent reports, USP7 silencing has also been shown to increase steady-state p53 levels by promoting Mdm2 degradation. More recently, both upregulation and downregulation of USP7 have been shown to inhibit colon cancer cell proliferation *in vitro* and tumor growth *in vivo*, by resulting in constitutively high p53 levels (Becker et al. Cell Cycle 2008, 7(9),1205-13.

USP7 has also been shown in human cells to deubiquitinate the well-known tumor suppressor gene PTEN, which provokes its nuclear export and hence its inactivation (Song et al., Nature 2008, Aug. 20). More importantly, USP7 overexpression was reported for the first time in prostate cancer and this overexpression was directly associated with tumour aggressiveness (Song et al., Nature 2008, Aug. 20).

USP7 has also been shown in human cells to deubiquitinate FOX04, which provokes its nuclear export and hence its inactivation; consequently the oncogenic PI3K/PKB signaling pathway was activated (van der Horst et al., Nat Cell Biol. 2006, 8, 1064-1073) Finally, USP7 plays an important role in p53-mediated cellular responses to various types of stress, such as DNA damage and oxidative stress (Marchenko et al., Embo J. 2007 26, 923-934), Meulmeester et al. Mol Cell 2005, 18, 565-576., van der Horst et al., Nat Cell Biol. 2006, 8, 1064-1073).

Synthetic inhibitors of USP7 protein binding containing the polypeptide portion P¹-Gly-P³-Ser, where P¹ is a glutamic acid residue or an amino acid with a non polar side chain and P³ is a glycine residue or an amino acid with non polar side chain, have been reported (W02006072048).

The phenotypes associated with USP7 silencing and the known connections between USP7 and essential viral proteins and oncogenic pathways, such as the p53/Mdm2 and PI3K/PKB pathways, strongly suggest that targeting USP7 with small-molecule inhibitors may be beneficial in the treatment of cancers and viral diseases.

To date, no specific USP7 small molecule inhibitors seem to have been reported.

The skeleton of 1,5-dihydro-pyrrol-2-one is known in the literature. Nevertheless, it has never been disclosed nor suggested that derivatives bearing this scaffold can be specific inhibitors of USP7.

According to a first object, the present invention concerns a compound of formula (I): 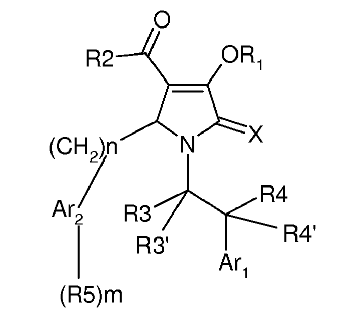 wherein :
Ar1 represents an aryl or heteroaryl group, said aryl or heteroaryl being optionally substituted by one or more substituent(s) chosen from : halogen atom, -alkyl, -aryl,
   - heterocycle, -alkylaryl, -CN, -NO₂, perfluoroalkyl-, perfluoroalkoxy-, polyfluoroalkyl-, polyfluoroalkoxy-, -OR, -NRR', =O, -C(=O)R, -C(=O)NRR',
   - C(=O)OR, -S(O)pR, -OC(=O)R; and/or said aryl or heteroaryl being optionally fused with an heterocyclic ring;
Ar2 represents an aryl or heteroaryl group, said aryl or heteroaryl,
   n is an integer chosen from 0 to 6;
   X is a O or S atom;
   R1 represents a hydrogen atom or an -alkyl group;
   R2 represents an alkyl, alkenyl, -alkylaryl, -alkylheteroaryl, -alkenylaryl, -Oalkyl, -NRR', cycloalkyl, aryl or heteroaryl group, said cycloalkyl, aryl or heteroaryl being optionally fused with a saturated or unsaturated cycloalkyl or heterocyclic ring and/or being optionally substituted by one or more substituent(s) chosen from : halogen atom, -alkyl;
   R3, R3', R4, R4' identical or different independently represent a hydrogen atom or an -alkyl, -aryl, -alkylaryl; and/or
wherein one of R3 or R3' forms a bond with an ortho-atom of Ar1 so as to form a cycloalkyl or heterocycle fused with Ar1 ;

Each R5 independently represents a halogen atom, -alkyl, -aryl, -alkylaryl, -CN, -NO₂, perfluoroalkyl-, perfluoroalkoxy-, polyfluoroalkyl-, polyfluoroalkoxy-, -OR, -NRR', =O, -C(=O)R, -C(=O)NRR', -C(=O)OR, -NRC(=O)R', -S(O)pR, -OC(=O)R, heteroaryl, heterocyclic ring, -O-alkyl-NRR',
and/or two R5 form with Ar2 to which they are attached a saturated or unsaturated cycloalkyl or heterocyclic ring, fused to Ar2,
and/or one R5 and R3 or R3' form a bond, so as to form a N-containing heterocyclic ring, and generate a tricyclic fused structure, comprising the pyrrolone, said form N-containing heterocycle and Ar2;
where R, R' identical or different, independently represent a hydrogen atom or an -alkyl, -aryl, -alkylaryl;
m is 0, or an integer chosen from 1 to 5;
p is 0, 1 or 2;
as well as its tautomers and its pharmaceutically acceptable salts.

Preferably, the present invention also concerns the following embodiments or any of their combination:
- Ar1 represents an aryl, preferably phenyl or naphtyl, or heteroaryl group, preferably thienyl, pyridyl, said aryl or heteroaryl being optionally substituted by one or more substituent(s) chosen from : halogen atom, -aryl, such as phenyl,
- OR, heterocycle, such as morpholinyl; and/or said aryl or heteroaryl being optionally fused with an heterocyclic ring, such as an oxiran; and/or
- R3=R3'=R4=R4'=H; and/or wherein one of R3 or R3' forms a bond with an ortho-atom of Ar1 so as to form a cycloalkyl, such as cylcopentyl, fused wit Ar1;
- Ar2 represents an aryl, preferably phenyl, or heteroaryl group, preferably pyridyl, said aryl or heteroaryl being optionally substituted by one or more substituent(s) chosen from : halogen atom, -alkyl, -aryl, -CN, -NO₂, perfluoroalkyl-, perfluoroalkoxy-, polyfluoroalkoxy-, -OR, -C(=O)OR, -NRC(=O)R', -S(O)pR, -OC(=O)R, heteroaryl, such as tetrazolyl, heterocyclic, preferably morpholinyl, ring, -O-alkyl-NRR'; and/or said aryl or heteroaryl being optionally fused with a saturated heterocyclic ring such as dioxolanyl;
- Ar2 is a phenyl group substituted on the para position;
- n is 0;
- X is a S atom;
- R1 represents a hydrogen atom or an -alkyl group, more preferably a hydrogen atom;
- R2 represents an alkyl, -alkenylaryl, -Oalkyl, cycloalkyl, aryl, such as phenyl, or heteroaryl group, such as pyridyl, said cycloalkyl, aryl or heteroaryl being optionally fused with a saturated heterocyclic ring such as dioxolanyl and/or being optionally substituted by one or more substituent(s) chosen from : halogen atom, -alkyl; more preferably R2 represents a -CHRR' group.

Preferred compounds of the invention are chosen from the group consisting in:
4-acetyl-5-(3,4-dichloro-phenyl)-3-hydroxy-1-phenethyl-1,5-dihydro-pyrrol-2-one
4-acetyl-3-hydroxy-1-phenethyl-5-(4-cyanophenyl)-1,5-dihydro-2H-pyrrol-2-one
4-Acetyl-5-(4-bromo-phenyl)-3-hydroxy-1-phenethyl-1,5-dihydro-pyrrol-2-one
4-acetyl-3-hydroxy-1-phenethyl-5-(4-acetoxyphenyl)-1,5-dihydro-2H-pyrrol-2-one
4-acetyl-3-hydroxy-1-phenethyl-5-(4-trifluoromethoxyphenyl)-1,5-dihydro-2H-pyrrol-2-one
4-acetyl-3-hydroxy-1-phenethyl-5-(4-difluoromethoxyphenyl)-1,5-dihydro-2H-pyrrol-2-one
4-acetyl-3-hydroxy-1-phenethyl-5-(4-butoxyphenyl)-1,5-dihydro-2H-pyrrol-2-one
4-Acetyl-3-hydroxy-1-phenethyl-5-(4-trifluoromethyl-phenyl)-1,5-dihydro-pyrrol-2-one
4-acetyl-3-hydroxy-1-phenethyl-5-(4-n-octyloxyphenyl)-1,5-dihydro-2H-pyrrol-2-one
4-acetyl-5-[4-(3-dimethylaminopropoxy)-phenyl]-3-hydroxy-1-phenethyl-1,5-dihydro-2H-pyrrol-2-one
4-acetyl-3-hydroxy-1-phenethyl-5-(4-morpholinylphenyl)-1,5-dihydro-2H-pyrrol-2-one
4-acetyl-5-(4-bromo-phenyl)-3-hydroxy-1-(2-pyridin-4-yl-ethyl)-1,5-dihydro-pyrrol-2-one
4-acetyl-3-hydroxy-1-phenethyl-5-[4-(1H-tetrazol-5-yl)-phenyl]-1,5-dihydro-2H-pyrrol-2-one
4-acetyl-3-hydroxy-5-(4-isopropoxyphenyl)-1-phenethyl-1,5-dihydro-2H-pyrrol-2-one
4-acetyl-5-(4-bromo-phenyl)-3-hydroxy-1-(2-thiophen-2-yl-ethyl)-1,5-dihydro-pyrrol-2-one
4-acetyl-5-(4-bromo-2-fluoro-phenyl)-3-hydroxy-1-phenethyl-1,5-dihydro-2H-pyrrol-2-one
4-acetyl-1-(2-biphenyl-4-yl-ethyl)-5-(4-bromo-phenyl)-3-hydroxy-1,5-dihydro-pyrrol-2-one
4-acetyl-5-(4-bromo-phenyl)-3-hydroxy-1-(3-phenyl-propyl)-1,5-dihydro-pyrrol-2-one
4-acetyl-5-(4-bromo-phenyl)-3-hydroxy-1-(4-phenyl-butyl)-1,5-dihydro-pyrrol-2-one
4-(3-acetyl-4-hydroxy-5-oxo-1-phenethyl-2,5-dihydro-1H-pyrrol-2-yl)-benzoicacid
4-acetyl-3-hydroxy-5-(4-methanesulfonyl-phenyl)-1-phenethyl-1,5-dihydro-2H-pyrrol-2-one
4-acetyl-3-hydroxy-1-phenethyl-5-pyridin-2-yl-1,5-dihydro-2H-pyrrol-2-one
4-acetyl-3-hydroxy-1-phenethyl-5-(4-hydroxy-phenyl)-1,5-dihydro-2H-pyrrol-2-one 4-acetyl-5-(4-tert-butyl-phenyl)-3-hydroxy-1-phenethyl-1,5-dihydro-2H-pyrrol-2-one
4-acetyl-5-(4-bromo-phenyl)-1-[2-(4-chloro-phenyl)-ethyl]-3-hydroxy-1,5-dihydro-pyrrol-2-one
4-acetyl-5-(4-bromo-phenyl)-1-[2-(4-bromo-phenyl)-ethyl]-3-hydroxy-1,5-dihydro-pyrrol-2-one
4-acetyl-5-biphenyl-4-yl-3-hydroxy-1-phenethyl-1,5-dihydro-2H-pyrrol-2-one
4-acetyl-5-(4-bromo-phenyl)-1-[2-(3,4-dichloro-phenyl)-ethyl]-3-hydroxy-1,5-dihydro-pyrrol-2-one
5-(4-bromo-phenyl)-4-cyclopropanecarbonyl-3-hydroxy-1-phenethyl-1,5-dihydro-pyrrol-2-one
4-acetyl-5-(4-bromo-phenyl)-3-hydroxy-1-[2-(3-methoxy-phenyl)-ethyl]-1,5-dihydro-pyrrol-2-one
4-acetyl-5-(4-bromo-phenyl)-3-hydroxy-1-[2-(2-methoxy-phenyl)-ethyl]-1,5-dihydro-pyrrol-2-one
4-acetyl-5-(4-bromo-phenyl)-3-hydroxy-1-[2-(4-methoxy-phenyl)-ethyl]-1,5-dihydro-pyrrol-2-one
4-acetyl-5-(4-bromo-phenyl)-3-hydroxy-1-[2-(4-fluorophenyl)-ethyl]-1,5-dihydro-pyrrol-2-one
4-acetyl-5-(4-bromo-phenyl)-3-hydroxy-1-[2-(4-ethoxy-phenyl)-ethyl]-1,5-dihydro-pyrrol-2-one
4-acetyl-3-hydroxy-1-phenethyl-5-pyridin-4-yl-1,5-dihydro-pyrrol-2-one
N-[4-(3-acetyl-4-hydroxy-5-oxo-1-phenethyl-2,5-dihydro-1H-pyrrol-2-yl)-phenyl]-acetamide
4-acetyl-5-benzo[1,3]dioxol-5-yl-3-hydroxy-1-phenethyl-1,5-dihydro-pyrrol-2-one
4-acetyl-3-hydroxy-1-phenethyl-5-pyridin-3-yl-1,5-dihydro-pyrrol-2-one
4-acetyl-5-benzo[1,3]dioxol-5-yl-1-[2-(3,4-dimethoxy-phenyl)-ethyl]-3-hydroxy-1,5-dihydro-pyrrol-2-one
4-acetyl-5-(4-chloro-3-fluoro-phenyl)-3-hydroxy-1-phenethyl-1,5-dihydro-pyrrol-2-one
4-acetyl-5-(3,4-dibromo-phenyl)-3-hydroxy-1-phenethyl-1,5-dihydro-pyrrol-2-one
4-acetyl-5-(2-chloro-pyridin-3-yl)-3-hydroxy-1-phenethyl-1,5-dihydro-pyrrol-2-one
4-acetyl-3-hydroxy-1-phenethyl-5-(6-trifluoromethyl-pyridin-3-yl)-1,5-dihydro-pyrrol-2-one
4-Acetyl-5-(4-bromo-phenyl)-1-[2-(2-chloro-phenyl)-ethyl]-3-hydroxy-1,5-dihydro-pyrrol-2-one
4-Acetyl-5-(4-chlorophenyl)-1-[2-(4-bromophenyl)-ethyl]-3-hydroxy-1,5-dihydro-pyrrol-2-one
4-Acetyl-5-(4-chlorophenyl)-1-[2-(4-chlorophenyl)-ethyl]-3-hydroxy-1,5-dihydro-pyrrol-2-one
4-Acetyl-5-(4-bromophenyl)-1-[2-(2,4-dichlorophenyl)-ethyl]-3-hydroxy-1,5-dihydro-pyrrol-2-one
4-acetyl-5-(4-bromo-3-chloro-phenyl)-3-hydroxy-1-phenethyl-1,5-dihydro-pyrrol-2-one
4-acetyl-3-hydroxy-5-(3-hydroxy-4-methoxy-phenyl)-1-phenethyl-1,5-dihydro-pyrrol-2-one
4-acetyl-1-(2-benzo[1,3]dioxol-5-yl-ethyl)-5-(4-chloro-phenyl)-3-hydroxy-1,5-dihydro-pyrrol-2-one
4-acetyl-5-(4-bromo-phenyl)-3-hydroxy-1-[2-(3-trifluoromethyl-phenyl)-ethyl]-1,5-dihydro-pyrrol-2-one
4-acetyl-5-(4-bromo-phenyl)-3-hydroxy-1-indan-2-yl-1,5-dihydro-pyrrol-2-one
4-acetyl-5-(4-bromophenyl)-1-[2-(3-bromophenyl)-ethyl]-3-hydroxy-1,5-dihydro-pyrrol-2-one
4-acetyl-5-(4-bromophenyl)-3-hydroxy-1-(2-m-tolyl-ethyl)-1,5-dihydro-pyrrol-2-one
4-Acetyl-5-(4-bromo-phenyl)-3-hydroxy-1-[2-(4-morpholin-4-yl-phenyl)-ethyl]-1,5-dihydro-pyrrol-2-one
4-acetyl-5-(4-bromophenyl)-3-hydroxy-1-(2-naphthalen-1-yl-ethyl)-1,5-dihydro-pyrrol-2-one
   as well as their tautomers and their pharmaceutically acceptable salts.

As used hereabove or hereafter:
"Alkyl" means an aliphatic hydrocarbon group which may be straight or branched having 1 to 20 carbon atoms in the chain. Preferred alkyl groups have 1 to 12 carbon atoms in the chain. "Branched" means that one or more lower alkyl groups such as methyl, ethyl or propyl are attached to a linear alkyl chain. Exemplary alkyl groups include methyl, ethyl, *n*-propyl, *i*-propyl, *n*-butyl, *t*-butyl, *n*-pentyl, 3-pentyl, octyl, nonyl, decyl.

As used herein, the term "cycloalkyl" refers to an aromatic or non aromatic hydrocarbon mono, bi or multi cyclic ring of 3 to 10 carbon atoms formed by the removal of one hydrogen atom. A designation such as "C₅-C₇ cycloalkyl" refers to a cycloalkyl radical containing from 5 to 7 carbon atoms. Examples include cyclopropyl, cyclopentyl, cyclohexyl, cycloheptyl, adamantyl, etc. as well as the systems formed by their condensation or by the condensation with a phenyl group.

"Alken" means an aliphatic hydrocarbon group containing a carbon-carbon double bond and which may be straight or branched having 2 to 15 carbon atoms in the chain. Preferred alkenyl groups have 2 to 12 carbon atoms in the chain; and more preferably about 2 to 4 carbon atoms in the chain. Exemplary alkenyl groups include ethenyl, propenyl, *n*-butenyl, *i*-butenyl, 3-methylbut-2-enyl, *n*-pentenyl, heptenyl, octenyl, nonenyl, decenyl.

-"Halogen atom" refers to fluorine, chlorine, bromine or iodine atom; preferably fluorine and chlorine atom.

"Perhalogenoalkyl" refers to an alkyl group as defined above where all H atoms are replaced by halogen atoms.

"Polyhalogenoalkyl" refers to an alkyl group as defined above where one or more H atoms are replaced by halogen atoms.

"Aryl" means an aromatic monocyclic or multicyclic hydrocarbon ring system of 6 to 14 carbon atoms, preferably of 6 to 10 carbon atoms. Exemplary aryl groups include phenyl or naphthyl.

As used herein, the terms "heterocycle" or "heterocyclic" refer to a saturated, partially unsaturated or unsaturated, non aromatic stable 3 to 14, preferably 5 to 10 membered mono, bi or multicyclic rings wherein at least one member of the ring is a hetero atom. Typically, heteroatoms include, but are not limited to, oxygen, nitrogen, sulfur, selenium, and phosphorus atoms. Preferable heteroatoms are oxygen, nitrogen and sulfur.

Suitable heterocycles are also disclosed in The Handbook of Chemistry and Physics, 76th Edition, CRC Press, Inc., 1995-1996, p. 2-25 to 2-26, the disclosure of which is hereby incorporated by reference.

Preferred non aromatic heterocyclic include, but are not limited to pyrrolidinyl, pyrazolidinyl, imidazolidinyl, oxiranyl, tetrahydrofuranyl, dioxolanyl, tetrahydro-pyranyl, dioxanyl, dioxolanyl, piperidyl, piperazinyl, morpholinyl, pyranyl, imidazolinyl, pyrrolinyl, pyrazolinyl, thiazolidinyl, tetrahydrothiopyranyl, dithianyl, thiomorpholinyl, dihydro-pyranyl, tetrahydropyranyl, dihydropyranyl, tetrahydro-pyridyl, dihydropyridyl, tetrahydropyrinidinyl, dihydrothiopyranyl, azepanyl, as well as the fused systems resulting from the condensation with a phenyl group.

As used herein, the term "heteroaryl" or aromatic heterocycles refers to a 5 to 14, preferably 5 to 10-membered aromatic hetero, mono-, bi- or multicyclic ring. Examples include pyrrolyl, pyridyl, pyrazolyl, thienyl, pyrimidinyl, pyrazinyl, tetrazolyl, indolyl, quinolinyl, purinyl, imidazolyl, thienyl, thiazolyl, benzothiazolyl, furanyl, benzofuranyl, 1,2,4-thiadiazolyl, isothiazolyl, triazoyl, tetrazolyl, isoquinolyl, benzothienyl, isobenzofuryl, pyrazolyl, carbazolyl, benzimidazolyl, isoxazolyl, pyridyl-N-oxide , as well as the fused systems resulting from the condensation with a phenyl group.

"Alkyl", "alkenyl", "cycloalkyl", "aryl", "heteroaryl", "heterocycle" and the likes refers also to the corresponding "alkylene", "alkenylene", "cycloalkylene", "arylene", "heteroarylene", "heterocyclene" and the likes which are formed by the removal of two hydrogen atoms.

As used herein, the term "patient" refers to either an animal, such as a valuable animal for breeding, company or preservation purposes, or preferably a human or a human child, which is afflicted with, or has the potential to be afflicted with one or more diseases and conditions described herein.

As used herein, a "therapeutically effective amount" refers to an amount of a compound of the present invention which is effective in preventing, reducing, eliminating, treating or controlling the symptoms of the herein-described diseases and conditions. The term "controlling" is intended to refer to all processes wherein there may be a slowing, interrupting, arresting, or stopping of the progression of the diseases and conditions described herein, but does not necessarily indicate a total elimination of all disease and condition symptoms, and is intended to include prophylactic treatment.

As used herein, the expression "pharmaceutically acceptable" refers to those compounds, materials, excipients, compositions or dosage forms which are, within the scope of sound medical judgment, suitable for contact with the tissues of human beings and animals without excessive toxicity, irritation, allergic response or other problem complications commensurate with a reasonable benefit/risk ratio.

As used herein, "pharmaceutically acceptable salts" refer to derivatives of the disclosed compounds wherein the parent compound is modified by making acid or base salts thereof. The pharmaceutically acceptable salts include the conventional non-toxic salts or the quaternary ammonium salts of the parent compound formed, for example, from non-toxic inorganic or organic acids. For example, such conventional non-toxic salts include those derived from inorganic acids such as hydrochloric, hydrobromic, sulfuric, sulfamic, phosphoric, nitric and the like; and the salts prepared from organic acids such as acetic, propionic, succinic, tartaric, citric, methanesulfonic, benzenesulfonic, glucoronic, glutamic, benzoic, salicylic, toluenesulfonic, oxalic, fumaric, maleic, lactic and the like. Further addition salts include ammonium salts such as tromethamine, meglumine, epolamine, etc., metal salts such as sodium, potassium, calcium, zinc or magnesium.

The pharmaceutically acceptable salts of the present invention can be synthesized from the parent compound which contains a basic or acidic moiety by conventional chemical methods. Generally, such salts can be prepared by reacting the free acid or base forms of these compounds with a stoichiometric amount of the appropriate base or acid in water or in an organic solvent, or in a mixture of the two. Generally, non-aqueous media like ether, ethyl acetate, ethanol, isopropanol, or acetonitrile are preferred. Lists of suitable salts are found in Remington's Pharmaceutical Sciences, 17th ed., Mack Publishing Company, Easton, PA, 1985, p. 1418, the disclosure of which is hereby incorporated by reference.

The compounds of the general formula (I) having geometrical and stereoisomers are also a part of the invention.

Further, the compounds of formula (I) may also have one or more tautomers. In particular, tautomers of formula (I) may include:

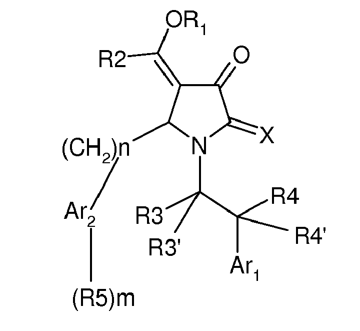

Tautomers of compounds of formula (I) are also part of the invention.

According to a further object, the present invention is also concerned with the process of preparation of the compounds of formula (I).

The compounds and process of the present invention may be prepared in a number of ways well-known to those skilled in the art. The compounds can be synthesized, for example, by application or adaptation of the methods described below, or variations thereon as appreciated by the skilled artisan. The appropriate modifications and substitutions will be readily apparent and well known or readily obtainable from the scientific literature to those skilled in the art.

In particular, such methods can be found in R.C. Larock, Comprehensive Organic Transformations, Wiley-VCH Publishers, 1999.

It will be appreciated that the compounds of the present invention may contain one or more asymmetrically substituted carbon atoms, and may be isolated in optically active or racemic forms. Thus, all chiral, diastereomeric, racemic forms, isomeric forms of a structure are intended, unless the specific stereochemistry or isomeric form is specifically indicated. It is well-known in the art how to prepare and isolate such optically active forms. For example, mixtures of stereoisomers may be separated by standard techniques including, but not limited to, resolution of racemic forms, normal, reverse-phase, and chiral chromatography, preferential salt formation, recrystallization, and the like, or by chiral synthesis either from chiral starting materials or by deliberate synthesis of target chiral centers.

Additionally, the process of the invention may lead to several regioisomers which are all encompassed by the present invention. Regioisomers are generally isolated by chromatography.

Compounds of the present invention may be prepared by a variety of synthetic routes. The reagents and starting materials are commercially available, or readily synthesized by well-known techniques by one of ordinary skill in the arts. All substituents, unless otherwise indicated, are as previously defined.

In the reactions described hereinafter, it may be necessary to protect reactive functional groups, for example hydroxyl, amino, imino, thio or carboxy groups, where these are desired in the final product, to avoid their unwanted participation in the reactions. Conventional protecting groups may be used in accordance with standard practice, for examples see T.W. Greene and P. G. M. Wuts in Protective Groups in Organic Chemistry, 3rd ed., John Wiley and Sons, 1999; J. F. W. McOmie in Protective Groups in Organic Chemistry, Plenum Press, 1973.

Some reactions may be carried out in the presence of a base. There is no particular restriction on the nature of the base to be used in this reaction, and any base conventionally used in reactions of this type may equally be used here, provided that it has no adverse effect on other parts of the molecule. Examples of suitable bases include: sodium hydroxide, potassium carbonate, triethylamine, alkali metal hydrides, such as sodium hydride and potassium hydride; alkyllithium compounds, such as methyllithium and butyllithium; and alkali metal alkoxides, such as sodium methoxide and sodium ethoxide.

Usually, reactions are carried out in a suitable solvent. A variety of solvents may be used, provided that it has no adverse effect on the reaction or on the reagents involved. Examples of suitable solvents include: hydrocarbons, which may be aromatic, aliphatic or cycloaliphatic hydrocarbons, such as hexane, cyclohexane, benzene, toluene and xylene; amides, such as dimethylformamide; alcohols such as ethanol and methanol and ethers, such as diethyl ether and tetrahydrofuran.

The reactions can take place over a wide range of temperatures. In general, it is found convenient to carry out the reaction at a temperature of from 0°C to 150°C (more preferably from about room temperature to 100°C). The time required for the reaction may also vary widely, depending on many factors, notably the reaction temperature and the nature of the reagents. However, provided that the reaction is effected under the preferred conditions outlined above, a period of from 3 hours to 20 hours will usually suffice.

The compound thus prepared may be recovered from the reaction mixture by conventional means. For example, the compounds may be recovered by distilling off the solvent from the reaction mixture or, if necessary, after distilling off the solvent from the reaction mixture, pouring the residue into water followed by extraction with a water-immiscible organic solvent and distilling off the solvent from the extract. Additionally, the product can, if desired, be further purified by various well-known techniques, such as recrystallization, reprecipitation or the various chromatography techniques, notably column chromatography or preparative thin layer chromatography.

The process of preparation of a compound of formula (I) of the invention is a further object of the present invention.

According to a first aspect, compounds of the invention of formula (I) can be obtained by reacting corresponding compounds of formula (II), (III) and (IV): 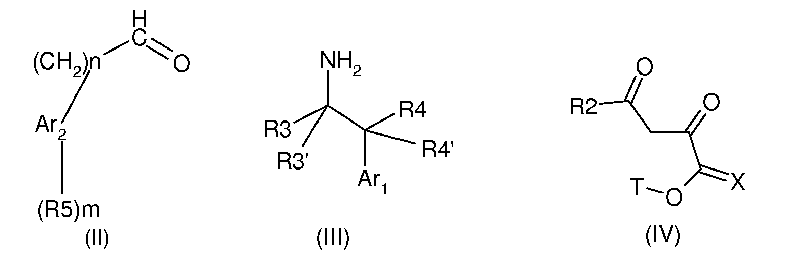 wherein R2, R3, R3', R4, R4', R5, Ar1, Ar2, X, m, n are defined as in formula (I) and T represents a alkyl group optionally substituted by one or more substituent(s) chosen from : halogen atom, -alkyl, -aryl, -CN, -NO₂, perfluoroalkyl-.

Generally, the reaction is carried out in an organic solvent, such as an alcohol (preferably ethanol), or in glacial acetic acid. The mixture may be reacted under stirring at a temperature comprised between room temperature and the boiling temperature of the reaction mixture.

Preferably, when using ethanol, compounds of formula (II) and (III) are first mixed and reacted, followed by addition of the compound of formula (IV). The reaction may be advantageously conducted at room temperature.

Preferably, when using glacial acetic acid, compound (II) is first reacted with glacial acetic acid, followed by the addition of compound of formula (III) and then (IV).

Compounds of formula (VI) may be obtained from corresponding compounds of formula (II), (IV) as discussed above and (III') : 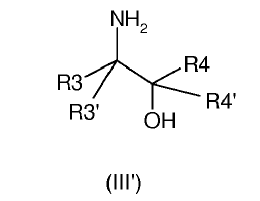 where R3, R3', R4, R4' are defined as in formula (I), by applying the same procedure as that for obtaining compounds of formula (I) from (II), (III) and (IV).

The above reactions can be carried out by the skilled person by applying or adapting the methods illustrated in the examples hereinafter.

Further, the process of the invention may also comprise the additional step of isolating the compound of formula (I). This can be done by the skilled person by any of the known conventional means, such as the recovery methods described above.

Generally, the starting products (II), (III), (III') and (IV) are commercially available mainly from Aldrich or Acros or other typical chemicals supplier or may be obtained by applying or adapting any known methods or those described in the

### examples.

For instance, the aldehyde (II) may be obtained by oxidizing the corresponding alcohol of formula (V): 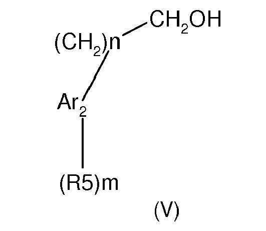 where Ar2, n, m, R5 are defined as in formula (II).This reaction may be carried out by applying standard oxidation methods. Preferably, compound (V) may be reacted in an aprotic organic solvent, such as dichloromethane, with manganese IV dioxide as an oxidizing agent.

The synthesis may also be carried out in one pot as a multicomponent reaction.

According to a further object, the present invention concerns also the pharmaceutical compositions comprising a compound of formula (I) as defined below: 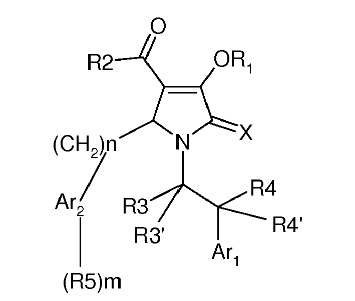 wherein :
Ar1 represents an aryl or heteroaryl group, said aryl or heteroaryl being optionally substituted by one or more substituent(s) chosen from : halogen atom, -alkyl, -aryl,
   - heterocycle, -alkylaryl, -CN, -NO₂, perfluoroalkyl-, perfluoroalkoxy-, polyfluoroalkyl-, polyfluoroalkoxy-, -OR, -NRR', =O, -C(=O)R, -C(=O)NRR',
   - C(=O)OR, -S(O)pR, -OC(=O)R; and/or said aryl or heteroaryl being optionally fused with an heterocyclic ring;
Ar2 represents an aryl or heteroaryl group, said aryl or heteroaryl,
   n is an integer chosen from 0 to 6;
   XisaOorSatom;
   R1 represents a hydrogen atom or an -alkyl group;
   R2 represents an alkyl, alkenyl, -alkylaryl, -alkylheteroaryl, -alkenylaryl, -Oalkyl, -NRR', cycloalkyl, aryl or heteroaryl group, said cycloalkyl, aryl or heteroaryl being optionally fused with a saturated or unsaturated cycloalkyl or heterocyclic ring and/or being optionally substituted by one or more substituent(s) chosen from : halogen atom, -alkyl;
   R3, R3', R4, R4' identical or different idependently represent a hydrogen atom or an -alkyl, -aryl, -alkylaryl; and/or
   Wherein one of R3 or R3' forms a bond with an ortho-atom of Ar1 so as to form a cycloalkyl or heterocycle fused wit Ar1 ;

Each R5 independently represents a halogen atom, -alkyl, -aryl, -alkylaryl, -CN, -NO₂, perfluoroalkyl-, perfluoroalkoxy-, polyfluoroalkyl-, polyfluoroalkoxy-, -OR, -NRR', =O, -C(=O)R, -C(=O)NRR', -C(=O)OR, -NRC(=O)R', -S(O)pR, -OC(=O)R, heteroaryl, heterocyclic ring, -O-alkyl-NRR',
and/or two R5 form with Ar2 to which they are attached a saturated or unsaturated cycloalkyl or heterocyclic ring, fused to Ar2,
and/or one R5 and R3 or R3' form a bond, so as to form a N-containing heterocyclic ring, and generate a tricyclic fused structure, comprising the pyrrolone, said form N-containing heterocycle and Ar2;
where R, R' identical or different, independently represent a hydrogen atom or an -alkyl, -aryl, -alkylaryl;
m is 0, or an integer chosen from 1 to 5;
p is 0, 1 or 2;
with a pharmaceutically acceptable excipient.

Preferred embodiments of formula (I) are as defined above in respect of the compounds of the invention.

According to a still further object, the present invention concerns a compound of formula (I): 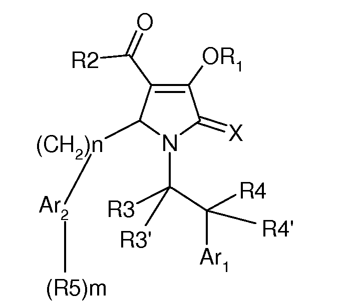 wherein :
Ar1 represents an aryl or heteroaryl group, said aryl or heteroaryl being optionally substituted by one or more substituent(s) chosen from : halogen atom, -alkyl, -aryl, -heterocycle, -alkylaryl, -CN, -NO₂, perfluoroalkyl-, perfluoroalkoxy-, polyfluoroalkyl-, polyfluoroalkoxy-, -OR, -NRR', =O, -C(=O)R, -C(=O)NRR', -C(=O)OR, -S(O)pR, -OC(=O)R; and/or said aryl or heteroaryl being optionally fused with an heterocyclic ring;
   Ar2 represents an aryl or heteroaryl group, said aryl or heteroaryl,
   n is an integer chosen from 0 to 6;
   X is a O or S atom;
   R1 represents a hydrogen atom or an -alkyl group;
   R2 represents an alkyl, alkenyl, -alkylaryl, -alkylheteroaryl, -alkenylaryl, -Oalkyl, -NRR', cycloalkyl, aryl or heteroaryl group, said cycloalkyl, aryl or heteroaryl being optionally fused with a saturated or unsaturated cycloalkyl or heterocyclic ring and/or being optionally substituted by one or more substituent(s) chosen from : halogen atom, -alkyl;
   R3, R3', R4, R4' identical or different idependently represent a hydrogen atom or an -alkyl, -aryl, -alkylaryl; and/or
wherein one of R3 or R3' forms a bond with an ortho-atom of Ar1 so as to form a cycloalkyl or heterocycle fused wit Ar1 ;

Each R5 independently represent a halogen atom, -alkyl, -aryl, -alkylaryl, -CN, -NO₂, perfluoroalkyl-, perfluoroalkoxy-, polyfluoroalkyl-, polyfluoroalkoxy-, -OR, -NRR', =O, -C(=O)R, -C(=O)NRR', -C(=O)OR, -NRC(=O)R', -S(O)pR, -OC(=O)R, heteroaryl, heterocyclic ring, -O-alkyl-NRR',
and/or two R5 form with Ar2 to which they are attached a saturated or unsaturated cycloalkyl or heterocyclic ring, fused to Ar2,
and/or one R5 and R3 or R3' form a bond, so as to form a N-containing heterocyclic ring, and generate a tricyclic fused structure, comprising the pyrrolone, said form N-containing heterocycle and Ar2;
where R, R' identical or different, independently represent a hydrogen atom or an -alkyl, -aryl, -alkylaryl;
m is 0, or an integer chosen from 1 to 5;
p is 0, 1 or 2;
for inhibiting cysteine protease.

The compounds of the invention are useful for inhibiting cysteine proteases, in particular specific de-ubiquitination enzymes such as USPs, and more particularly USP-7 in patients in the need thereof.

The compounds of the invention are particularly useful for treating and/or preventing cancer and metastasis, more particularly prostate and/or colon cancers, neurodegenerative diseases such as Alzheimer's disease and Parkinson's disease, deafness, disorders associated with ageing; viral acute or latent infections by Herpes simplex virus-1, Epstein-Barr virus, SARS coronavirus, rhinoviruses, poliomyelitis virus, hepatitis A virus, hepatitis C virus, adenoviruses, and the like.

The present invention also concerns the corresponding methods of treatment comprising the administration of a compound of the invention together with a pharmaceutically acceptable carrier or excipient to a patient in the need thereof.

The identification of those subjects who are in need of treatment of herein-described diseases and conditions is well within the ability and knowledge of one skilled in the art. A veterinarian or a physician skilled in the art can readily identify, by the use of clinical tests, physical examination, medical/family history or biological and diagnostic tests, those subjects who are in need of such treatment.

A therapeutically effective amount can be readily determined by the attending diagnostician, as one skilled in the art, by the use of conventional techniques and by observing results obtained under analogous circumstances. In determining the therapeutically effective amount, a number of factors are considered by the attending diagnostician, including, but not limited to: the species of subject; its size, age, and general health; the specific disease involved; the degree of involvement or the severity of the disease; the response of the individual subject; the particular compound administered; the mode of administration; the bioavailability characteristic of the preparation administered; the dose regimen selected; the use of concomitant medication; and other relevant circumstances.

The amount of a compound of formula (I), which is required to achieve the desired biological effect, will vary depending upon a number of factors, including the chemical characteristics (e.g. hydrophobicity) of the compounds employed, the potency of the compounds, the type of disease, the species to which the patient belongs, the diseased state of the patient, the route of administration, the bioavailability of the compound by the chosen route, all factors which dictate the required dose amounts, delivery and regimen to be administered.

"Pharmaceutically" or "pharmaceutically acceptable" refer to molecular entities and compositions that do not produce an adverse, allergic or other untoward reaction when administered to an animal, or a human, as appropriate.

As used herein, "pharmaceutically acceptable excipient" includes any carriers, diluents, adjuvants, or vehicles, such as preserving or antioxidant agents, fillers, disintegrating agents, wetting agents, emulsifying agents, suspending agents, solvents, dispersion media, coatings, antibacterial and antifungal agents, isotonic and absorption delaying agents and the like. The use of such media and agents for pharmaceutical active substances is well-known in the art. Except insofar as any conventional media or agent is incompatible with the active ingredient, its use in the therapeutic compositions is contemplated. Supplementary active ingredients can also be incorporated into the compositions as suitable therapeutic combinations.

In the context of the invention, the term "treating" or "treatment", as used herein, means reversing, alleviating, inhibiting the progress of, or preventing the disorder or condition to which such term applies, or one or more symptoms of such disorder or condition.

"Therapeutically effective amount" means an amount of a compound/medicament according to the present invention effective in preventing or treating a pathological condition requiring the inhibition of an active cysteine protease involved in its pathogenesis.

According to the invention, the terms "patient" or "patient in need thereof", are intended for an animal or a human being affected or likely to be affected with a pathological condition involving an active cysteine protease in its pathogenesis. Preferably, the patient is human.

In general terms, the compounds of this invention may be provided in an aqueous physiological buffer solution containing 0.1 to 10 % w/v compound for parenteral administration. Typical dose ranges are from 1 µg/kg to 0.1 g/kg of body weight per day; a preferred dose range is from 0.01 mg/kg to 10 mg/kg of body weight per day or an equivalent dose in a human child. The preferred dosage of drug to be administered is likely to depend on such variables as the type and extent of progression of the disease or disorder, the overall health status of the particular patient, the relative biological efficacy of the compound selected, the formulation of the compound, the route of administration (intravenous, intramuscular, or other), the pharmacokinetic properties of the compound by the chosen delivery route, and the speed (bolus or continuous infusion) and schedule of administrations (number of repetitions in a given period of time).

The compounds of the present invention are also capable of being administered in unit dose forms, wherein the expression "unit dose" means a single dose which is capable of being administered to a patient, and which can be readily handled and packaged, remaining as a physically and chemically stable unit dose comprising either the active compound itself, or as a pharmaceutically acceptable composition, as described hereinafter. As such, typical total daily dose ranges are from 0.01 to 100 mg/kg of body weight. By way of general guidance, unit doses for humans range from 1 mg to 3000 mg per day. Preferably, the unit dose range is from 1 to 500 mg administered one to six times a day, and even more preferably from 10 mg to 500 mg, once a day. Compounds provided herein can be formulated into pharmaceutical compositions by admixture with one or more pharmaceutically acceptable excipients. Such unit dose compositions may be prepared for use by oral administration, particularly in the form of tablets, simple capsules or soft gel capsules; or intranasally, particularly in the form of powders, nasal drops, or aerosols; or dermally, for example, topically in ointments, creams, lotions, gels or sprays, or via trans-dermal patches.

The compositions may conveniently be administered in unit dosage form and may be prepared by any of the methods well-known in the pharmaceutical art, for example, as described in Remington: The Science and Practice of Pharmacy, 20th ed.; Gennaro, A. R., Ed.; Lippincott Williams & Wilkins: Philadelphia, PA, 2000.

Preferred formulations include pharmaceutical compositions in which a compound of the present invention is formulated for oral or parenteral administration.

For oral administration, tablets, pills, powders, capsules, troches and the like can contain one or more of any of the following ingredients, or compounds of a similar nature: a binder such as microcrystalline cellulose, or gum tragacanth; a diluent such as starch or lactose; a disintegrant such as starch and cellulose derivatives; a lubricant such as magnesium stearate; a glidant such as colloidal silicon dioxide; a sweetening agent such as sucrose or saccharin; or a flavoring agent such as peppermint, or methyl salicylate. Capsules can be in the form of a hard capsule or soft capsule, which are generally made from gelatin blends optionally blended with plasticizers, as well as a starch capsule. In addition, dosage unit forms can contain various other materials that modify the physical form of the dosage unit, for example, coatings of sugar, shellac, or enteric agents. Other oral dosage forms syrup or elixir may contain sweetening agents, preservatives, dyes, colorings, and flavorings. In addition, the active compounds may be incorporated into fast dissolve, modified-release or sustained-release preparations and formulations, and wherein such sustained-release formulations are preferably bi-modal. Preferred tablets contain lactose, cornstarch, magnesium silicate, croscarmellose sodium, povidone, magnesium stearate, or talc in any combination.

Liquid preparations for parenteral administration include sterile aqueous or non-aqueous solutions, suspensions, and emulsions. The liquid compositions may also include binders, buffers, preservatives, chelating agents, sweetening, flavoring and coloring agents, and the like. Non-aqueous solvents include alcohols, propylene glycol, polyethylene glycol, vegetable oils such as olive oil, and organic esters such as ethyl oleate. Aqueous carriers include mixtures of alcohols and water, buffered media, and saline. In particular, biocompatible, biodegradable lactide polymer, lactide/glycolide copolymer, or polyoxyethylene-polyoxypropylene copolymers may be useful excipients to control the release of the active compounds. Intravenous vehicles can include fluid and nutrient replenishers, electrolyte replenishers, such as those based on Ringer's dextrose, and the like. Other potentially useful parenteral delivery systems for these active compounds include ethylene-vinyl acetate copolymer particles, osmotic pumps, implantable infusion systems, and liposomes.

Alternative modes of administration include formulations for inhalation, which include such means as dry powder, aerosol, or drops. They may be aqueous solutions containing, for example, polyoxyethylene-9-lauryl ether, glycocholate and deoxycholate, or oily solutions for administration in the form of nasal drops, or as a gel to be applied intranasally. Formulations for buccal administration include, for example, lozenges or pastilles and may also include a flavored base, such as sucrose or acacia, and other excipients such as glycocholate. Formulations suitable for rectal administration are preferably presented as unit-dose suppositories, with a solid based carrier, such as cocoa butter, and may include a salicylate. Formulations for topical application to the skin preferably take the form of an ointment, cream, lotion, paste, gel, spray, aerosol, or oil. Carriers which can be used include petroleum jelly, lanolin, polyethylene glycols, alcohols, or their combinations. Formulations suitable for transdermal administration can be presented as discrete patches and can be lipophilic emulsions or buffered, aqueous solutions, dissolved and/or dispersed in a polymer or an adhesive.

The invention is further illustrated but not restricted by the description in the following examples.

### EXPERIMENTAL

Representative compounds of the invention can be synthesized according to the following procedures:

### General procedure 1

Under argon or nitrogen, a one-necked round bottomed flask was charged, with an acetopyruvate derivative (1 equiv.) in absolute ethanol or glacial acetic acid, the corresponding aldehyde (1 equiv.) and the corresponding amine (1 equiv.). The reaction mixture was stirred at ambient temperature for 30min to 4 days or heated for several hours. In most of the cases a precipitate appeared and was collected and washed with an appropriate solvent (Ethanol, ether), dried at 40°C under high vacuum yielding the corresponding 1,5-dihydro-pyrrol-2-one.

### PREPARATION A : benzo[1,3]dioxole-5-carbaldehyde or piperonal

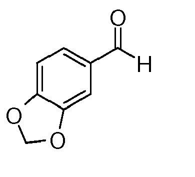

A 100 mL one-necked round bottomed flask was charged with piperonyl alcohol (1.03 g, 6.66 mmol) in dichloromethane (30 mL) and with manganese IV dioxide (6.43 g, 66.66 mmol). The reaction mixture was stirred at ambient temperature during 2 days. After evaporation of dichloromethane under reduced pressure, the suspension was filtered off on celite and washed with boiled dimethylformamide. After evaporation of the solvents under reduced pressure, the product crystallized and was dried under high vacuum yielding piperonal (983 mg, 98%) as yellow crystals.
¹H NMR (300 MHz, CHCl₃-d): δ 9.80 (s, 1H), 7.40 (m, 1H), 7.32 (m, 1H), 6.92 (d, 1H, *J*=7.9 Hz), 6.07 (s, 2H).
MS (ESI-) m/z: 184.2 (M+Na)⁺.

### PREPARATION B : 4-bromo-3-chloro-N-methoxy-N-methyl-benzamide

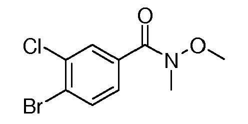

Under argon, a 10 mL one-necked round bottomed flask was charged with 4-bromo-3-chlorobenzoic acid (1.74 g, 7.18 mmol) in dry dimethylformamide (10 mL), followed by hydroxybenzotriazole (1.09 g, 7.90 mmol) and after 5 minutes stirring at ambient temperature, N-ethyl-N'-(3-dimethylaminopropyl)carbodiimide (1.54 g, 7.90 mmol). The reaction mixture was stirred at ambient temperature during 30 minutes. Then, at 0°C, N,O-dimethylhydroxylamine (0.80 g, 7.90 mmol) was poured. The reaction mixture was stirred at ambient temperature during 16 hours. The reaction mixture was co-evaporated with toluene in vacuo. The residue was diluted with sodium hydrogenocarbonate (40 mL) and the aqueous layer was extracted with ethyl acetate (3 x 40 mL). The organic layer was washed with water (2 x 30 mL), brine (30 mL), dried over anhydrous magnesium sulfate and evaporated in vacuo. The product was dried under high vacuum yielding 4-bromo-3-chloro-N-methoxy-N-methyl-benzamide (1.81 g, 91 %) as a yellow solid.
¹H NMR (300 MHz, DMSO-d₆): δ 7.85 (AB system, 1H, *J*=8.3 Hz), 7.80 (d, 1H, *J*=1.9 Hz), 7.48 (dd, 1H, *J*=1.9 Hz, 8.3 Hz), 3.55 (s, 3H), 3.26 (s, 3H).

### PREPARATION C : 4-bromo-3-chlorobenzaldehyde

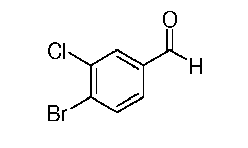

Under argon, at -78°C, a 10 mL one-necked round bottomed flask was charged with 4-bromo-3-chloro-N-methoxy-N-methyl-benzamide (preparation B) (1.80 g, 6.46 mmol) in dry tetrahydrofuran (32 mL). Then, a 1M solution of diisobutylaluminiumhydride (8.4 mL, 8.40 mmol) was then added dropwise. The reaction mixture was stirred at -78°C during 1 hour. The reaction mixture was quenched with water (50 mL) and the aqueous layer was extracted with ethyl acetate (3 x 50 mL). The organic layer was washed with a 1 N hydrochloric acid solution (30 mL), water (30 mL), brine (30 mL), dried over anhydrous magnesium sulfate and evaporated in vacuo. The product was dried under high vacuum yielding 4-bromo-3-chlorobenzaldehyde (1.18 g, 84%) as a yellow solid.
¹H NMR (300 MHz, DMSO-d₆): δ 9.98 (s, 1H), 8.11 (d, 1H, *J*=1.9 Hz), 8.02 (AB system, 1H, *J*=8.2 Hz), 7.77 (dd, 1H, *J*=1.9 Hz, 8.2 Hz).

### PREPARATION D : [2-(4-Bromo-phenyl)-ethyl]-carbamic acid tert-butyl ester

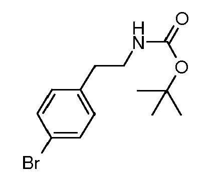

Under argon, a 100 mL one-necked round bottomed flask was charged with 4-bromophenethylamine (2.0 g, 10.0 mmol) in iPrOH (50.0 mL). (Boc)₂O (2.2 g, 10.0 mmol) was then added followed by triethylamine (2.8 mL, 20.0 mmol). The reaction mixture was stirred at ambient temperature during 2 hours. The reaction mixture was concentrated then diluted with a saturated solution of sodium hydrogenocarbonate and extracted with ethyl acetate. The organic layer was dried over anhydrous magnesium sulfate, filtered, evaporated in vacuo and dried under high vacuum yielding [2-(4-bromophenyl)-ethyl]-carbamic acid tert-butyl ester (3.4 g, quant.) as a white powder.
¹H NMR (300 MHz, CDCl₃-d): δ 7.42 (dd, 2H, *J*=6.6 Hz, *J*=1.7 Hz), 7.06 (d, 2H, *J*=8.3 Hz), 4.51 (bs, 1H), 3.33 (m, 2H), 2.75 (m, 2H), 1.43 (s, 9H).

### PREPARATION E : [2-(4-Morpholin-4-yl-phenyl)-ethyl] -carbamic acid tert-butyl ester

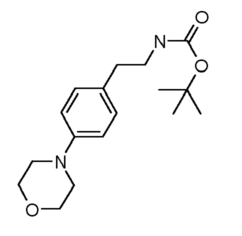

Under argon, a 10 mL one-necked round bottomed flask was charged with [2-(4-bromophenyl)-ethyl]-carbamic acid tert-butyl ester (Preparation D) (300 mg, 1.0 mmol) in dioxane (3.0 mL). Morpholine (218 µL, 2.5 mmol) was added followed by 2,2'-bis(diphenylphosphino)-1,1'-binaphtyl (124 mg, 0.2 mmol) and cesium carbonate (978 mg, 3.0 mmol). The reaction mixture was purged with argon during 10 minutes and palladium II acetate (23 mg, 0.1 mmol) was added. The reaction mixture was stirred at 110°C during 16 hours and diluted with dichloromethane. This mixture was absorbed on silica gel and purified by flash chromatography (Hex:EtOAc 100:0 to 0:100). The isolated compound was dried under high vacuum yielding [2-(4-morpholin-4-yl-phenyl)-ethyl]-carbamic acid tert-butyl ester (120 mg, 40%) as a pale brown powder.
¹H NMR (300 MHz, CDCl₃-d): δ 7.10 (AB system, 2H, *J*=8.6 Hz), 6.86 (AB system, 2H, *J*=8.6 Hz), 4.51 (bs, 1H), 3.86 (t, 4H, *J*=4.8 Hz), 3.33 (m, 2H), 3.13 (t, 4H, *J*=4.8 Hz), 2.72 (t, 2H, *J*=6.9 Hz), 1.43 (s, 9H). MS (ESI+) m/z: 307.38 (M+H)⁺.

### PREPARATION F : 2-(4-Morpholin-4-yl-phenyl)-ethylamine

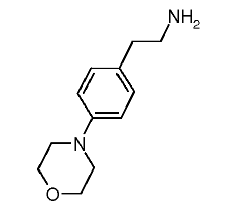

Under argon, a 10 mL one-necked round bottomed flask was charged with [2-(4-Morpholin-4-yl-phenyl)-ethyl]-carbamic acid tert-butyl ester (Preparation E) (120 mg, 0.39 mmol) in dioxane (1.0 mL). Concentrated HCI (1.0 mL) was added and the reaction mixture was stirred at ambient temperature overnight. The reaction was quenched with water, extracted with ethyl acetate. The organic phase was dried over anhydrous magnesium sulphate and evaporated in vacuo. The obtained compound was dried under high vacuum yielding 2-(4-morpholin-4-yl-phenyl)-ethylamine (75 mg, 93%) as a pale brown solid.
¹H NMR (300 MHz, CDCl₃-d): δ 7.11 (AB system, 2H, *J*=8.6 Hz), 6.86 (AB system, 2H, *J*=8.6 Hz), 3.86 (t, 4H, *J*=4.8 Hz), 3.13 (t, 4H, *J*=4.8 Hz), 2.93 (t, 2H, *J*=6.8 Hz), 2.68 (t, 2H, *J*=6.6 Hz).

### EXAMPLE 1 : 4-acetyl-5-(3,4-dichloro-phenyl)-3-hydroxy-1-phenethyl-1,5-dihydro-pyrrol-2-one

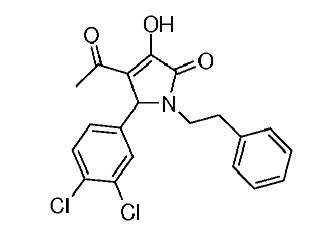

Under argon, a 10 mL one-necked round bottomed flask was charged with 3,4-dichlorobenzaldehyde (114 mg, 0.64 mmol) in absolute ethanol (3.2 mL) and with 2-phenylethylamine (82 µL, 0.64 mmol). The reaction mixture was stirred at ambient temperature during 1 hour. Then, ethyl acetopyruvate (93 µL, 0.64 mmol) was added to the solution. The reaction mixture was stirred at ambient temperature during 4 hours. A precipitation occurred after 2 hours stirring. The precipitate was collected and washed with a small amount of diethyl ether. The product was dried at 60°C under high vacuum yielding 31 mg of 4-acetyl-5-(3,4-dichloro-phenyl)-3-hydroxy-1-phenethyl-1,5-dihydro-pyrrol-2-one.
12%, white powder.
¹H NMR (300 MHz, DMSO-d₆): δ 7.55 (d, 2H, *J*=7.3 Hz), 7.44 (d, 1H, *J*=1.7 Hz), 7.29 - 7.21 (m, 3H), 7.17 - 7.09 (m, 3H), 5.11 (s, 1H), 3.79 (m, 1H), 2.79 (m, 2H),
2.69 (m, 1H), 2.27 (s, 3H).
MS (ESI-) m/z: 390.4 (M-H)⁻.

### EXAMPLE 2 : 4-acetyl-3-hydroxy-1-phenethyl-5-(4-cyanophenyl)-1,5-dihydro-2H-pyrrol-2-one

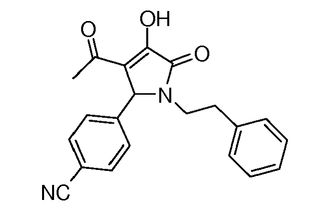

Under argon, a 10 mL one-necked round bottomed flask was charged with 4-cyanobenzaldehyde (83 mg, 0.62 mmol) in glacial acetic acid (0.6 mL). 2-phenylethylamine (79 µL, 0.62 mmol) was then added followed by addition of ethyl acetopyruvate (90 µL, 0.57 mmol). The reaction mixture was stirring at 95°C during 2 hours then at ambient temperature during 7 days until the product crystallized. The precipitate was collected and washed with diethyl ether (3x3 mL). The product was dried at 40°C under high vacuum yielding 72 mg of 4-acetyl-3-hydroxy-1-phenethyl-5-(4-cyanophenyl)-1,5-dihydro-2H-pyrrol-2-one.
33%, grey powder.
¹H NMR (300 MHz, CHCl₃-d): δ 7.62 (AB system, 1H, *J*=8.4 Hz), 7.33-7.23 (m, 3H), 7.14 (AB system, 1H, *J*=8.4 Hz), 7.10-7.07 (m, 2H), 4.79 (s, 1H), 4.02 (m, 1H), 2.84 (m, 3H), 2.16 (s, 3H).
MS (ESI+) m/z: 347.0 (M+H)⁺.

### EXAMPLE 3 : 4-Acetyl-5-(4-bromo-phenyl)-3-hydroxy-1-phenethyl-1,5-dihydro-pyrrol-2-one

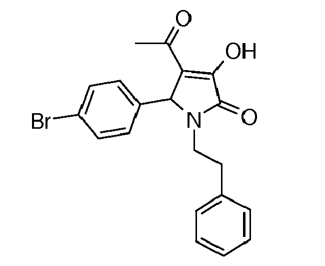

### Prepared using procedure I using :

Methylacetopyruvate, 4-bromobenzaldehyde and phenethylamine 46%, colourless solid.
¹H NMR (300 MHz, CHCl₃-d): δ 7.46 (AB system, 2H, *J*=8.3 Hz), 7.35-7.25 (m, 3H), 7.09 (d, 2H, *J*=6.4 Hz), 6.91 (AB sytem, 2H, *J*=8.3 Hz), 4.71 (s, 1H), 3.97 (m, 1H), 2.93 - 2.80 (m, 2H), 2.79 - 2.70 (m, 1H), 2.03 (s, 3H).
MS (ESI+) m/z: m/z: 399.9 (M+H)⁺.

### EXAMPLE 4 : 4-acetyl-3-hydroxy-1-phenethyl-5-(4-acetoxyphenyl)-1,5-dihydro-2H-pyrrol-2-one

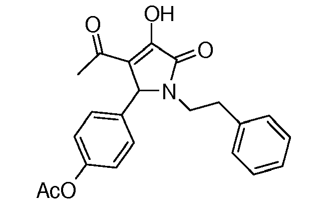

### Prepared using procedure I using :

4-acetoxybenzaldehyde (96 mg, 0.57 mmol) in absolute ethanol, 2-phenylethylamine and methyl acetopyruvate 25%, white powder.
¹H NMR (300 MHz, DMSO-d₆): δ 7.28-7.17 (m, 5H), 7.11-7.06 (m, 4H), 5.08 (s, 1H), 3.75 (m, 1H), 2.77 (m, 2H), 2.77 (m, 1H), 2.27 (s, 3H), 2.25 (s, 3H).
MS (ESI+) m/z: 380.0 (M+H)⁺.

### EXAMPLE 5 : 4-acetyl-3-hydroxy-1-phenethyl-5-(4-trifluoromethoxyphenyl)-1,5-dihydro-2H-pyrrol-2-one

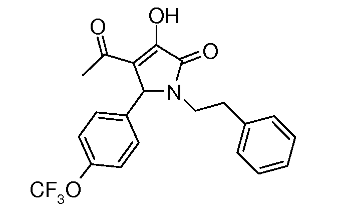

### Prepared using procedure I using :

4-(trifluoromethoxy)benzaldehyde, 2-phenylethylamine and ethyl acetopyruvate, 1h 24%, white powder.
¹H NMR (300 MHz, CHCl₃-d): δ 7.32-7.26 (m, 3H), 7.20-7.17 (m, 2H), 7.11-7.05 (m, 4H), 4.76 (s, 1H), 3.96 (m, 1H), 2.89 (m, 2H), 2.81 (m, 1H), 2.02 (s, 3H).
MS (ESI+) m/z: 405.9 (M+H)⁺.

### EXAMPLE 6 : 4-acetyl-3-hydroxy-1-phenethyl-5-(4-difluoromethoxyphenyl)-1,5-dihydro-2H-pyrrol-2-one

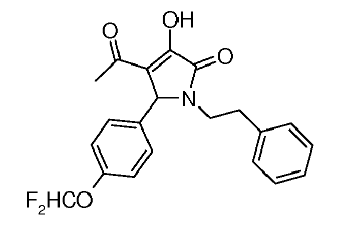

### Prepared using procedure I using :

4-(difluoromethoxy)benzaldehyde, 2-phenylethylamine and ethyl acetopyruvate 68%, white powder.
¹H NMR (300 MHz, CHCl₃-d): δ 7.32-7.24 and 7.11-7.02 (m, 9H), 6.51 (t, 1H, *J*=73.4 Hz), 4.75 (s, 1H), 3.96 (m, 1H), 2.89 (m, 2H), 2.77 (m, 1H), 2.00 (s, 3H).

### EXAMPLE 7 : 4-acetyl-3-hydroxy-1-phenethyl-5-(4-butoxyphenyl)-1,5-dihydro-2H-pyrrol-2-one

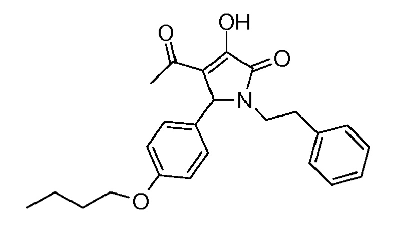

### Prepared using procedure I using :

4-(Butuloxy)benzaldehyde, 2-phenylethylamine, ethyl acetopyruvate ] 72%, white powder.
¹H NMR (300 MHz, CHCl₃-d): δ 7.32-7.26 (m, 3H), 7.12-7.09 (m, 2H), 6.89 (AB system, 4H, *J*=8.7 Hz), 4.72 (s, 1H), 3.94 (t, 2H, *J*=6.4 Hz), 3.93 (m, 1H), 2.89 (m, 2H), 2.73 (m, 1H), 1.89 (s, 3H), 1.75 (m, 2H), 1.49 (m, 2H), 0.97 (t, 3H, *J*=7.3 Hz).
MS (ESI+) m/z: 394.0 (M+H)⁺.

### EXAMPLE 8 : 4-Acetyl-3-hydroxy-1-phenethyl-5-(4-trifluoromethyl-phenyl)-1,5-dihydro-pyrrol-2-one

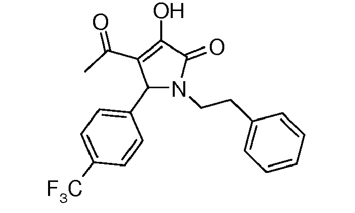

### Prepared using procedure I using :

4-(trifluoromethyl)benzaldehyde, 2-phenylethylamine and methyl acetopyruvate 48%, white powder.
¹H NMR (300 MHz, CHCl₃-d): δ 7.60 (d, 2H, *J*=8.1 Hz), 7.35-7.23 (m, 3H), 7.15 (d, 2H, *J*=8.0 Hz), 7.09 (m, 2H), 4.81 (s, 1H), 4.00 (m, 1H), 2.86 (m, 3H), 2.07 (s, 3H). MS (ESI+) m/z: 389.9 (M+H)⁺.

### EXAMPLE 9 : 4-acetyl-3-hydroxy-1-phenethyl-5-(4-n-octyloxyphenyl)-1,5-dihydro-2H-pyrrol-2-one

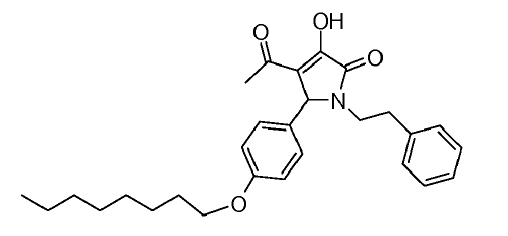

### Prepared using procedure I using :

4-(n-octyloxy)benzaldehyde, 2-phenylethylamine and ethyl acetopyruvate 46%, white powder.
¹H NMR (300 MHz, CHCl₃-d): δ 7.31-7.23 (m, 3H), 7.10 (d, 2H, *J*=6.6 Hz), 6.95 (AB system, 1H, *J*=8.7 Hz), 6.85 (AB system, 1H, *J*=8.7 Hz), 4.73 (s, 1H), 3.93 (t, 2H, *J*=6.5 Hz), 3.90 (m, 1H), 2.88 (m, 2H), 2.73 (m, 1H), 1.93 (s, 3H), 1.77 (m, 2H), 1.44 (m, 2H), 1.28 (m, 8H), 0.86 (bt, 3H).
MS (ESI+) m/z: 450.6 (M+H)⁺.

### EXAMPLE 10 : 4-acetyl-5-[4-(3-dimethylaminopropoxy)-phenyl]-3-hydroxy-1-phenethyl-1,5-dihydro-2H-pyrrol-2-one

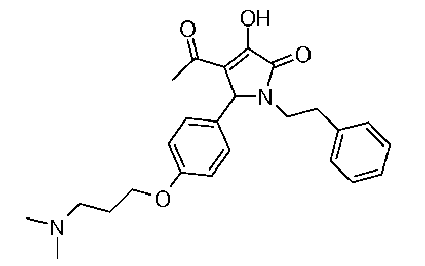

### Prepared using procedure I using :

4-(3-dimethylaminopropoxy)benzaldehyde, 2-phenylethylamine and ethyl acetopyruvate 99%, pink powder.
¹H NMR (300 MHz, CHCl₃-d): δ 7.23-7.14 (m, 3H), 7.06 (m, 2H), 7.03 (AB system, 1H, *J*=8.6 Hz), 6.74 (AB system, 1H, *J*=8.6 Hz), 4.88 (s, 1H), 4.03 (t, 2H, *J*=6.1 Hz), 3.75 (m, 1H), 3.19 (m, 3H), 2.79 (s, 6H), 2.72 (m, 2H), 2.63 (m, 2H), 2.22 (m,
2H), 2.17 (s, 3H).
MS (ESI+) m/z: 423.2 (M+H)⁺.

### EXAMPLE 11 : 4-acetyl-3-hydroxy-1-phenethyl-5-(4-morpholinylphenyl)-1,5-dihydro-2H-pyrrol-2-one

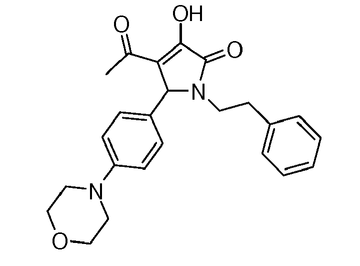

### Prepared using procedure I using :

4-(morpholinyl)benzaldehyde, 2-phenylethylamine and ethyl acetopyruvate 19%, pink powder.
¹H NMR (300 MHz, CHCl₃-d): δ 7.31-7.23 (m, 3H), 7.11 (m, 2H), 6.93 (AB system, 2H, *J*=8.6 Hz), 6.86 (AB system, 2H, *J*=8.6 Hz), 4.71 (s, 1H), 3.93 (m, 1H), 3.86 (m, 4H), 3.17 (m, 4H), 2.89 (m, 2H), 2.74 (m, 1H), 1.91 (s, 3H).
MS (ESI+) m/z: 407.8 (M+H)⁺.

### EXAMPLE 12 : 4-acetyl-5-(4-bromo-phenyl)-3-hydroxy-1-(2-pyridin-4-yl-ethyl)-1,5-dihydro-pyrrol-2-one

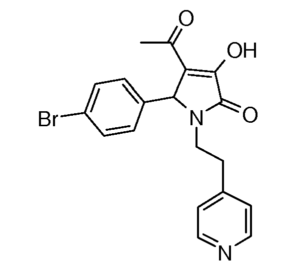

### Prepared using procedure I using :

4-bromo benzaldehyde, 4-(2-aminoethyl)pyridine and ethyl acetopyruvate 65%, white powder.
¹H NMR (300 MHz, DMSO-d₆): δ8.43 (dd, 2H, *J*=4.5 Hz, *J*=1.4 Hz), 7.51 (AB system, 2H, *J*=8.4 Hz), 7.16 (m, 4H), 5.17 (s, 1H), 3.83 (m, 1H), 2.77 (m, 3H), 2.25 (s, 3H).
MS (ESI+) m/z: 400.9 (M+H)⁺.

### EXAMPLE 13 : 4-acetyl-3-hydroxy-1-phenethyl-5-[4-(1H-tetrazol-5-yl)-phenyl]-1,5-dihydro-2H-pyrrol-2-one

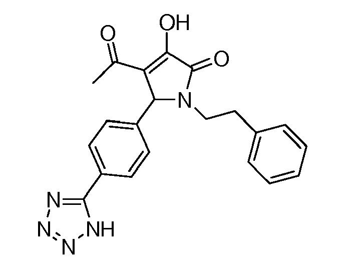

### Prepared using procedure I using :

4-(1H-tetrazol-5-yl)-benzaldehyde, 2-phenylethylamine and ethyl acetopyruvate 9%, white powder.
¹H NMR (300 MHz, CHCl₃-d): δ 7.99 (AB system, 2H, *J*=7.5 Hz), 7.38 (AB system, 2H, *J*=7.5 Hz), 7.26-7.10 (m, 5H), 5.15 (s, 1H), 3.82 (m, 1H), 2.80-2.70 (m, 3H), 2.27 (s, 3H).
MS (ESI+) m/z: 390.0 (M+H)⁺.

### EXAMPLE 14 : 4-acetyl-3-hydroxy-5-(4-isopropoxyphenyl)-1-phenethyl-1,5-dihydro-2H-pyrrol-2-one

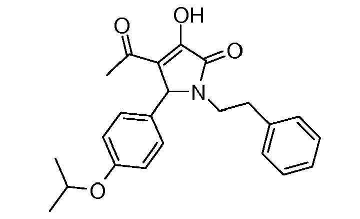

### Prepared using procedure I using :

4-isopropoxybenzaldehyde, 2-phenylethylamine and ethyl acetopyruvate 84%, white powder.
¹H NMR (300 MHz, CHCl₃-d): δ 7.29-7.16 (m, 3H), 7.10 (d, 2H, *J*=6.8 Hz), 7.02 (AB system, 2H, *J*=8.6 Hz), 6.83 (AB system, 2H, *J*=8.6 Hz), 4.99 (s, 1H), 4.57 (sextuplet, 1H, *J*=6.0 Hz), 3.74 (m, 1H), 2.76 (m, 2H), 2.60 (m, 1H), 2.25 (s, 3H), 1.24 (d, 6H, *J*=6.0 Hz).
MS (ESI+) m/z: 380.0 (M+H)⁺.

### EXAMPLE 15 : 4-acetyl-5-(4-bromo-phenyl)-3-hydroxy-1-(2-thiophen-2-yl-ethyl)-1,5-dihydro-pyrrol-2-one

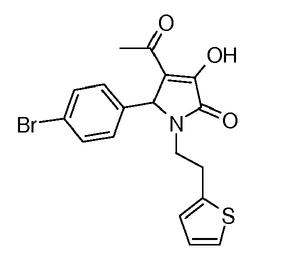

### Prepared using procedure I using :

4-bromo benzaldehyde, thiophene-2-ethylamine and ethyl acetopyruvate 52%, white powder.
¹H NMR (300 MHz, CHCl₃-d): δ 7.48 (AB system, 2H, *J*=8.4 Hz), 7.18 (dd, 1H, *J*=1.1 Hz, 5.1 Hz), 6.97 (AB system, 2H, *J*=8.4 Hz), 6.94 (m, 1H), 6.78 (d, 1H, *J*=3.3 Hz), 4.79 (s, 1H), 3.97 (m, 1H), 3.11 (m, 1H), 3.05-2.85 (m, 2H), 2.05 (s,
3H).
MS (ESI+) m/z: 407.8 (M+H)⁺.

### EXAMPLE 16 : 4-acetyl-5-(4-bromo-2-fluoro-phenyl)-3-hydroxy-1-phenethyl-1,5-dihydro-2H-pyrrol-2-one

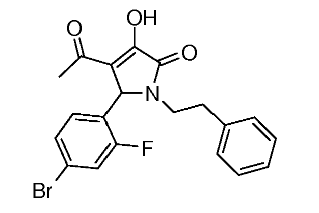

### Prepared using procedure I using :

4-bromo-2-fluoro-benzaldehyde, 2-phenylethylamine and ethyl acetopyruvate 26%, white powder.
¹H NMR (300 MHz, CHCl₃-d): δ 7.50 (d, 1H, *J*=9.8 Hz), 7.33-7.15 (m, 4H), 7.09 (m, 2H), 7.01 (m, 1H), 5.24 (s, 1H), 3.77 (m, 1H), 2.80 (m, 2H), 2.74 (m, 1H), 2.19 (s, 3H).
MS (ESI+) m/z: 418.0 (M+H)⁺.

### EXAMPLE 17 : 4-acetyl-1-(2-biphenyl-4-yl-ethyl)-5-(4-bromo-phenyl)-3-hydroxy-1,5-dihydro-pyrrol-2-one

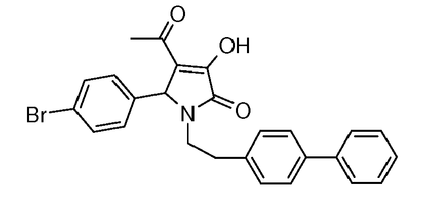

### Prepared using procedure I using :

4-bromo benzaldehyde, 2-(4-biphenyl)ethylamine and ethyl acetopyruvate 60 %, pale brown powder.
¹H NMR (300 MHz, CHCl₃-d): δ7.62-7.40 (m, 8H), 7.35 (m, 1H), 7.17 (AB system, 2H, *J*=8.4 Hz), 6.95 (AB system, 2H, *J*=8.4 Hz), 4.83 (s, 1H), 4.01 (m, 1H), 3.00-2.72 (m, 3H), 2.02 (s, 3H).
MS (ESI+) m/z: 476.1 (M+H)⁺.

### EXAMPLE 18 : 4-acetyl-5-(4-bromo-phenyl)-3-hydroxy-1-(3-phenyl-propyl)-1,5-dihydro-pyrrol-2-one

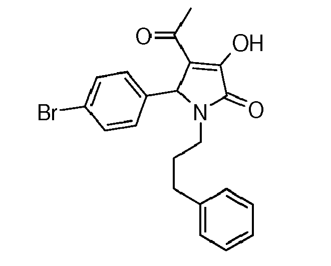

### Prepared using procedure I using :

4-bromobenzaldehyde, 3-phenyl-1-propylamine and ethyl-2,4-dioxovalerate 76%, white powder.
¹H NMR (300 MHz, CHCl₃-d): δ 7.46 (AB system, 2H, *J*=8.5 Hz), 7.26 (m, 2H), 7.17 (m, 1H), 7.09 (m, 2H), 7.00 (d, 2H, *J*=8.4 Hz), 5.02 (s, 1H), 3.74 (m, 1H), 2.74 (m, 1H), 2.56 (t, 2H, *J*=7.5 Hz), 2.10 (s, 3H), 1.80 (m, 2H).
MS (ESI+) m/z: 413.9 (M+H)⁺.

### EXAMPLE 19 : 4-acetyl-5-(4-bromo-phenyl)-3-hydroxy-1-(4-phenyl-butyl)-1,5-dihydro-pyrrol-2-one

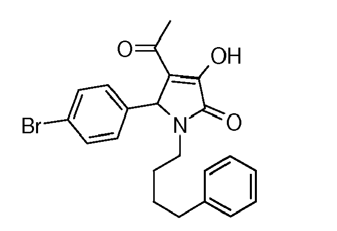

### Prepared using procedure I using :

4-bromobenzaldehyde, 4-phenylbutylamine and ethyl-2,4-dioxovalerate 66%, pale yellow powder.
¹H NMR (300 MHz, CHCl₃-d): δ 7.48 (AB system, 2H, *J*=8.3 Hz), 7.25 (m, 2H), 7.20 (m, 1H), 7.11 (d, 2H, *J*= 7.1 Hz), 7.00 (d, 2H, *J*=8.2 Hz), 5.00 (s, 1H), 3.75 (m, 1H), 2.75 - 2.45 (m, 3H), 2.10 (s, 3H), 1.53 (m, 4H).
MS (ESI+) m/z: 428.0 (M+H)⁺.

### EXAMPLE 20 : 4-(3-acetyl-4-hydroxy-5-oxo-1-phenethyl-2,5-dihydro-1H-pyrrol-2-yl)-benzoic acid

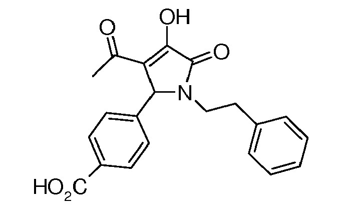

### Prepared using procedure I using :

4-carboxybenzaldehyde, 2-phenylethylamine and ethyl acetopyruvate 58%, white powder.
¹H NMR (300 MHz, DMSO-d₆): δ 12.96 (bs, 1H), 7.88 (AB system, 2H, *J*=8.0 Hz), 7.28-7.18 (m, 5H), 7.10 (AB system, 2H, *J*=7.0 Hz), 5.13 (s, 1H), 3.79 (m, 1H), 2.78 - 2.61 (m, 3H), 2.26 (s, 3H).
MS (ESI+) m/z: 366.0 (M+H)⁺.

### EXAMPLE 21 : 4-acetyl-3-hydroxy-5-(4-methanesulfonyl-phenyl)-1-phenethyl-1,5-dihydro-2H-pyrrol-2-one

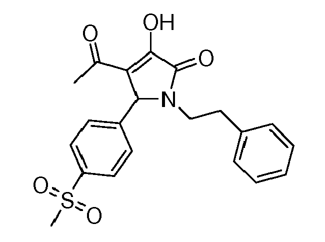

### Prepared using procedure I using :

4-methylsulfonylbenzaldehyde, 2-phenylethylamine and ethyl acetopyruvate 46%, white powder.
¹H NMR (300 MHz, DMSO-d₆): δ 7.86 (AB system, 2H, *J*=8.3 Hz), 7.42 (AB system, 2H, *J*=8.3 Hz), 7.28 - 7.19 (m, 3H), 7.11 (AB system, 2H, *J*=6.8 Hz), 5.17 (s, 1H), 3.80 (m, 1H), 3.22 (s, 3H), 2.82 - 2.64 (m, 3H), 2.26 (s, 3H).
MS (ESI+) m/z: 400.0 (M+H)⁺.

### EXAMPLE 22 : 4-acetyl-3-hydroxy-1-phenethyl-5-pyridin-2-yl-1,5-dihydro-2H-pyrrol-2-one

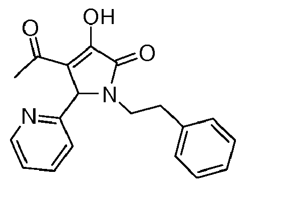

### Prepared using procedure I using :

2-pyridine-carboxaldehyde, 2-phenylethylamine and ethyl acetopyruvate 82%, white powder.
¹H NMR (300 MHz, CHCl₃-d): δ 8.60 (m, 1H), 7.72 (m, 1H), 7.34 - 7.09 (m, 7H), 5.20 (s, 1H), 4.00 (m, 1H), 2.93 (m, 2H), 2.74 (m, 1H), 2.16 (s, 3H).
MS (ESI+) m/z: 323.1 (M+H)⁺.

### EXAMPLE 23 : 4-acetyl-3-hydroxy-1-phenethyl-5-(4-hydroxy-phenyl)-1,5-dihydro-2H-pyrrol-2-one

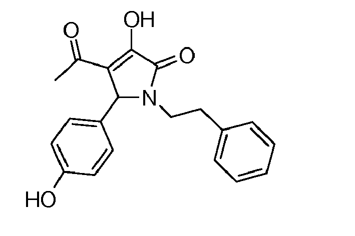

### Prepared using procedure I using :

4-hydroxybenzaldehyde, 2-phenylethylamine and ethyl acetopyruvate 20%, white powder.
¹H NMR (300 MHz, DMSO-d₆): δ 9.45, (s, 1H), 7.26 - 7.19 (m, 3H), 7.10 (d, 2H, *J*=7.0 Hz), 6.91 (AB system, 2H, *J*=8.0 Hz), 6.69 (AB system, 2H, *J*=8.0 Hz), 4.95 (s, 1H), 3.73 (m, 1H), 2.76 (m, 2H), 2.74 (m, 1H), 2.24 (s, 3H).
MS (ESI+) m/z: 338.0 (M+H)⁺.

### EXAMPLE 24: 4-acetyl-5-(4-tert-butyl-phenyl)-3-hydroxy-1-phenethyl-1,5-dihydro-2H-pyrrol-2-one

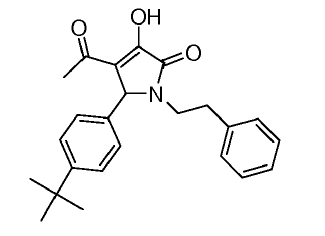

### Prepared using procedure I using :

4-tert-butyl-benzaldehyde, 2-phenylethylamine and ethyl acetopyruvate 50%, white powder.
¹H NMR (300 MHz, CHCl₃-d): δ 7.35 (AB system, 2H, *J*=8.3 Hz), 7.29 - 7.23 (m, 3H), 7.10 (d, 2H, *J*=6.5 Hz), 6.98 (AB system, 2H, *J*=8.3 Hz), 4.75 (s, 1H), 3.94 (m, 1H), 2.90 (m, 2H), 2.71 (m, 1H), 1.92 (s, 3H), 1.30 (s, 9H).
MS (ESI+) m/z: 378.1 (M+H)⁺.

### EXAMPLE 25 : 4-acetyl-5-(4-bromo-phenyl)-1-[2-(4-chloro-phenyl)-ethyl]-3-hydroxy-1,5-dihydro-pyrrol-2-one

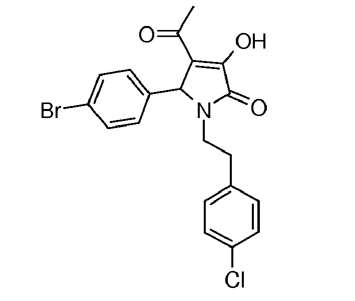

### Prepared using procedure I using :

4-bromobenzaldehyde, 2-(4-chlorophenyl)ethylamine and ethyl-2,4-dioxovalerate 23%, white powder.
¹H NMR (300 MHz, CHCl₃-d): δ 7.49 (AB system, 2H, *J*=8.3 Hz), 7.26 (m, 2H), 7.02 (d, 2H, *J*=8.3 Hz), 6.95 (d, 2H, *J*=8.3 Hz), 4.83 (s, 1H), 3.90 (m, 1H), 2.98 - 2.65 (m, 3H), 2.05 (s, 3H).
MS (ESI+) m/z: 433.9 (M+H)⁺.

### EXAMPLE 26: 4-acetyl-5-(4-bromo-phenyl)-1-[2-(4-bromo-phenyl)-ethyl]-3-hydroxy-1,5-dihydro-pyrrol-2-one

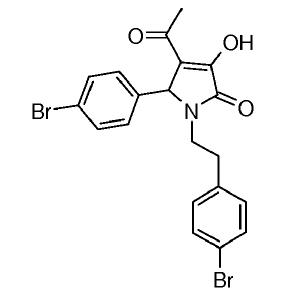

### Prepared using procedure I using :

4-bromobenzaldehyde, 2-(4-bromophenyl)ethylamine and ethyl-2,4-dioxovalerate 6%, white powder.
¹H NMR (300 MHz, CHCl₃-d): δ 7.49 (d, 2H, *J*=8.2 Hz), 7.40 (d, 2H, *J*=8.1 Hz), 6.96 (d, 4H, *J*=6.3 Hz), 4.83 (s, 1H), 3.90 (m, 1H), 2.98 - 2.65 (m, 3H), 2.04 (s, 3H).
MS (ESI+) m/z: 479.8 (M+H)⁺.

### EXAMPLE 27 : 4-acetyl-5-biphenyl-4-yl-3-hydroxy-1-phenethyl-1,5-dihydro-2H-pyrrol-2-one

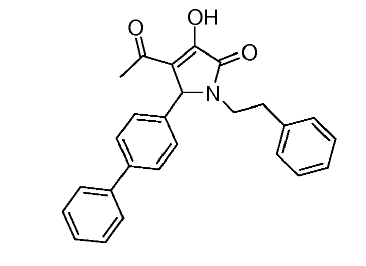

### Prepared using procedure I using :

4-biphenyl-carboxaldehyde, 2-phenylethylamine and ethyl acetopyruvate 14%, white powder.
¹H NMR (300 MHz, DMSO-d₆): δ 7.60-7.11 (m, 14H), 5.08 (s, 1H), 3.80 (m, 1H), 2.80 - 2.72 (m, 3H), 3.20 (s, 3H).
MS (ESI+) m/z: 398.0 (M+H)⁺.

### EXAMPLE 28 : 4-acetyl-5-(4-bromo-phenyl)-1-[2-(3,4-dichloro-phenyl)-ethyl]-3-hydroxy-1,5-dihydro-pyrrol-2-one

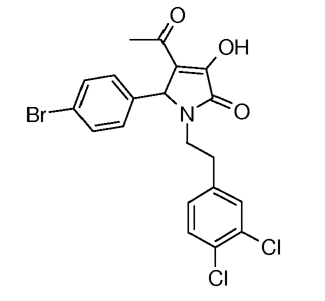

### Prepared using procedure I using :

4-bromobenzaldehyde, 3,4-dichlorophenethylamine and ethyl-2,4-dioxovalerate 30%, white powder.
¹H NMR (300 MHz, CHCl₃-d): δ 7.51 (AB system, 2H, *J*=8.4 Hz), 7.34 (AB system, 1H, *J*=8.2 Hz), 7.16 (d, 1H, *J*=1.7 Hz), 6.98 (AB system, 2H, *J*=8.3 Hz), 6.92 (dd, 1H, *J*=1.9 Hz, 8.2 Hz), 4.89 (s, 1H), 3.86 (m, 1H), 2.98 (m, 1H), 2.88 - 2.65 (m, 2H), 2.07 (s, 3H).
MS (ESI+) m/z: 469.9 (M+H)⁺.

### EXAMPLE 29 : 5-(4-bromo-phenyl)-4-cyclopropanecarbonyl-3-hydroxy-1-phenethyl-1,5-dihydro-pyrrol-2-one

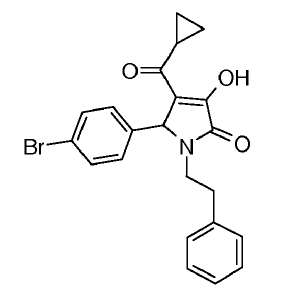

### Prepared using procedure I using :

4-bromobenzaldehyde, phenethylamine and 4-cyclopropyl-2,4-dioxobutyric acid methyl ester 50%, white powder.
¹H NMR (300 MHz, CHCl₃-d): δ 7.47 (AB system, 2H, *J*=8.4 Hz), 7.28 (m, 3H), 7.11 (m, 2H), 6.93 (AB system, 2H, *J*= 8.4 Hz), 4.86 (s, 1H), 3.97 (m, 1H), 2.88 (m, 2H), 2.75 (m, 1H), 1.75 (m, 1H), 1.11 (m, 1H), 0.98 (m, 1H), 0.90 (m, 1H), 0.70 (m, 1H).
MS (ESI+) m/z: 426.0 (M+H)⁺.

### EXAMPLE 30 : 4-acetyl-5-(4-bromo-phenyl)-3-hydroxy-1-[2-(3-methoxyphenyl)-ethyl]-1,5-dihydro-pyrrol-2-one

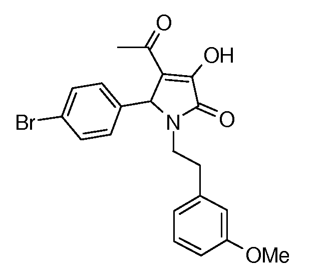

### Prepared using procedure I using :

4-bromobenzaldehyde, 3-methoxyphenethylamine and ethyl-2,4-dioxovalerate 13%, white powder.
¹H NMR (300 MHz, CHCl₃-d): δ 7.48 (AB system, 2H, *J*=8.2 Hz), 7.21 (m, 1H), 6.92 (AB system, 2H, *J*=8.2 Hz), 6.79 (d, 1H, *J*=6.2 Hz), 6.68 (d, 1H, *J*=7.5 Hz), 6.62 (s, 1H), 4.75 (s, 1H), 3.97 (m, 1H), 3.78 (s, 3H), 2.88 (m, 2H), 2.75 (m, 1H), 2.01 (s, 3H).
MS (ESI+) m/z: 430.0 (M+H)⁺.

### EXAMPLE 31 : 4-acetyl-5-(4-bromo-phenyl)-3-hydroxy-1-[2-(2-methoxyphenyl)-ethyl]-1,5-dihydro-pyrrol-2-one

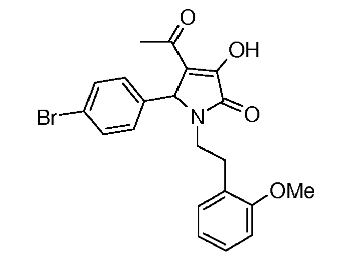

### Prepared using procedure I using :

4-bromobenzaldehyde, 2-methoxyphenethylamine and ethyl-2,4-dioxovalerate 61 %, white powder.
¹H NMR (300 MHz, CDCl₃-d): δ 7.45 (AB system, 2H, *J*=8.4 Hz), 7.23 (m, 1H), 7.05 (dd, 1H, *J*=1.4 Hz, 7.3 Hz), 6.89 (AB system, 2H, *J*=8.4 Hz), 6.85 (m, 1H), 4.80 (s, 1H), 3.94 (m, 1H), 3.73 (s, 3H), 2.88 (m, 3H), 2.04 (s, 3H).
MS (ESI+) m/z: 430.0 (M+H)⁺.

### EXAMPLE 32 : 4-acetyl-5-(4-bromo-phenyl)-3-hydroxy-1-[2-(4-methoxyphenyl)-ethyl]-1,5-dihydro-pyrrol-2-one

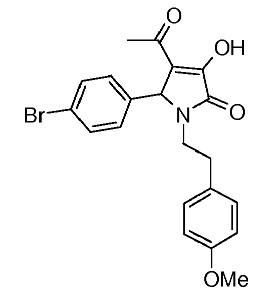

### Prepared using procedure I using :

4-bromobenzaldehyde, 4-methoxyphenethylamine and ethyl-2,4-dioxovalerate 26%, white powder.
¹H NMR (300 MHz, CDCl₃-d): δ 7.48 (AB system, 2H, *J*=8.4 Hz), 7.01 (AB system, 2H, *J*=8.6 Hz), 6.93 (AB system, 2H, *J*=8.4 Hz), 6.83 (AB system, 2H, *J*=8.6 Hz), 4.78 (s, 1H), 3.94 (m, 1H), 3.79 (s, 3H), 2.82 (m, 2H), 2.70 (m, 1H), 2.03 (s, 3H).
MS (ESI+) m/z: 430.0 (M+H)⁺.

### EXAMPLE 33 : 4-acetyl-5-(4-bromo-phenyl)-3-hydroxy-1-[2-(4-fluorophenyl)-ethyl]-1,5-dihydro-pyrrol-2-one

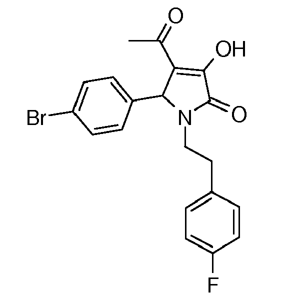

### Prepared using procedure I using :

4-bromobenzaldehyde, 4-fluorophenethylamine and ethyl-2,4-dioxovalerate 16%, white powder.
¹H NMR (300 MHz, CDCl₃-d): δ 7.49 (AB system, 2H, *J*=7.9 Hz), 7.10 - 6.85 (m, 6H), 4.81 (s, 1H), 3.90 (m, 1H), 2.85 (m, 2H), 2.73 (m, 1H), 2.04 (s, 3H).
MS (ESI+) m/z: 420.0 (M+H)⁺.

### EXAMPLE 34 : 4-acetyl-5-(4-bromo-phenyl)-3-hydroxy-1-[2-(4-ethoxy-phenyl)-ethyl]-1,5-dihydro-pyrrol-2-one

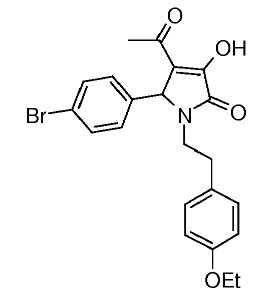

### Prepared using procedure I using :

4-bromobenzaldehyde, 4-ethoxyphenethylamine and ethyl-2,4-dioxovalerate 42%, white powder.
¹H NMR (300 MHz, CDCl₃-d): δ 7.48 (AB system, 2H, *J*=8.3 Hz), 6.99 (AB system, 2H, *J*=8.4 Hz), 6.93 (AB system, 2H, *J*=8.3 Hz), 6.82 (AB system, 2H, *J*=8.5 Hz), 4.77 (s, 1H), 4.01 (q, 2H, *J*=6.9 Hz), 3.92 (m, 1H), 2.81 (m, 2H), 2.70 (m, 1H), 2.03 (s, 3H), 1.41 (t, 3H, *J*=7.0 Hz).
MS (ESI+) m/z: 444.0 (M+H)⁺.

### EXAMPLE 35 : 4-acetyl-3-hydroxy-1-phenethyl-5-pyridin-4-yl-1,5-dihydro-pyrrol-2-one

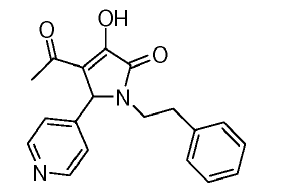

### Prepared using procedure I using :

4-pyridinecarboxaldehyde, 2-phenylethylamine and ethyl acetopyruvate 96%, yellow powder.
¹H NMR (300 MHz, DMSO-d₆): δ 8.51 (m, 2H), 7.26 - 7.10 (m, 7H), 5.08 (s, 1H), 3.81 (m, 1H), 2.77 - 2.64 (m, 3H), 2.27 (s, 3H).
MS (ESI+) m/z: 323.1 (M+H)⁺.

### EXAMPLE 36 : N-[4-(3-acetyl-4-hydroxy-5-oxo-1-phenethyl-2,5-dihydro-1H-pyrrol-2-yl)-phenyl]-acetamide

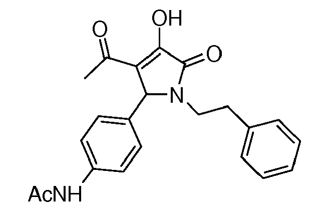

### Prepared using procedure I using :

4-acetamidobenzaldehyde, 2-phenylethylamine and ethyl acetopyruvate 88%, white powder.
¹H NMR (300 MHz, DMSO-d₆): δ 9.93 (s, 1H), 7.50 (AB system, 2H, *J*=8.3 Hz), 7.25 (AB system, 2H, *J*=8.3 Hz), 7.24 - 7.18 (m, 2H), 7.12 - 7.04 (m, 3H), 4.99 (s, 1H), 3.76 (m, 1H), 2.72 (m, 2H), 2.60 (m, 1H), 2.25 (s, 3H), 2.02 (s, 3H).
MS (ESI+) m/z: 379.0 (M+H)⁺.

### EXAMPLE 37 : 4-acetyl-5-benzo[1,3]dioxol-5-yl-3-hydroxy-1-phenethyl-1,5-dihydro-pyrrol-2-one

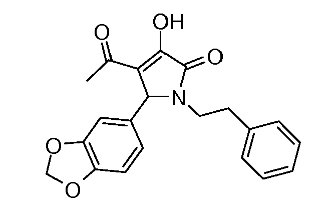

### Prepared using procedure I using :

Piperonal according to Preparation A, 2-phenylethylamine and ethyl acetopyruvate 40%, white powder.
¹H NMR (300 MHz, DMSO-d₆): δ 7.26 - 7.10 (m, 5H), 6.85 (m, 1H), 6.68 - 6.62 (m, 2H), 5.99 (s, 2H), 4.98 (s, 1H), 3.76 (m, 1H), 2.78 (m, 2H), 2.62 (m, 1H), 2.25 (s, 3H).
MS (ESI+) m/z: 366.0 (M+H)⁺.

### EXAMPLE 38: 4-acetyl-3-hydroxy-1-phenethyl-5-pyridin-3-yl-1,5-dihydro-pyrrol-2-one

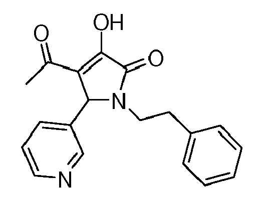

### Prepared using procedure I using :

3-pyridinecarboxaldehyde, 2-phenylethylamine and ethyl acetopyruvate 96%, white powder.
¹H NMR (300 MHz, DMSO-d₆): δ 8.48 (m, 2H), 7.47 (m, 1H), 7.34 - 7.19 (m, 4H), 7.17 - 7.10 (m, 2H), 5.11 (s, 1H), 3.80 (m, 1H), 2.79 (m, 2H), 2.65 (m, 1H), 2.27 (s, 3H).
MS (ESI+) m/z: 323.0 (M+H)⁺.

### EXAMPLE 39 : 4-acetyl-5-benzo[1,3]dioxol-5-yl-1-[2-(3,4-dimethoxy-phenyl)-ethyl]-3-hydroxy-1,5-dihydro-pyrrol-2-one

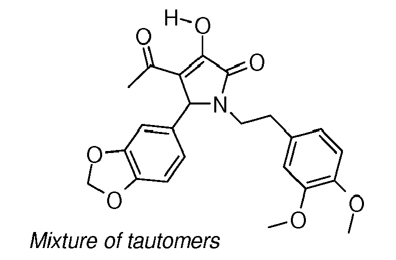

### Prepared using procedure I using :

Piperonal according to Preparation A, 3,4-dimethoxyphenethylamine and ethyl acetopyruvate
12%, pink powder.
¹H NMR (300 MHz, DMSO-d₆): δ 6.83 - 6.80 (m, 2H), 6.68 - 6.58 (m, 4H), 5.97 (bs, 2H), 4.93 (bs, 1H), 3.70 (bs, 6H), 3.69 (m, 1H), 2.72 (m, 3H), 2.07 (bs, 3H).
MS (ESI-) m/z: 426.1 (M+H)⁺.

### EXAMPLE 40 : 4-acetyl-5-(4-chloro-3-fluoro-phenyl)-3-hydroxy-1-phenethyl-1,5-dihydro-pyrrol-2-one

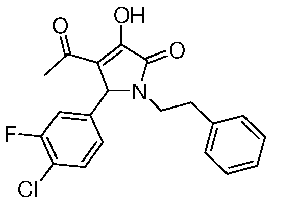

### Prepared using procedure I using :

4-chloro-3-fluorobenzaldehyde, 2-phenethylamine and ethyl acetopyruvate 6%, white powder.
¹H NMR (300 MHz, DMSO-d₆): δ 7.52 (dd, 1H, *J*=7.7 Hz, 7.9 Hz), 7.26 - 7.10 (m, 6H), 7.01 (bd, 1H, J=7.7 Hz ), 5.11 (s, 1H), 3.80 (m, 1H), 2.79 (m, 2H), 2.67 (m,
1H), 2.27 (s, 3H).
MS (ESI-) m/z: 373.9 (M+H)⁺.

### EXAMPLE 41 : 4-acetyl-5-(3,4-dibromo-phenyl)-3-hydroxy-1-phenethyl-1,5-dihydro-pyrrol-2-one

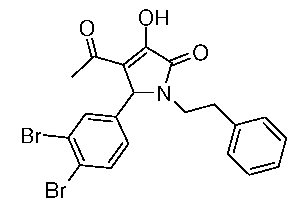

### Prepared using procedure I using :

3,4-dibromobenzaldehyde, 2-phenethylamine and ethyl acetopyruvate 21 %, white powder.
¹H NMR (300 MHz, DMSO-d₆): δ 7.67 (d, 1H, *J*=8.2 Hz), 7.56 (d, 1H, *J*=1.9 Hz ), 7.29 - 7.17 (m, 3H), 7.12 (dd, 2H, *J*=1.9 Hz, 8.2 Hz), 7.03 (m, 1H), 5.08 (s, 1H), 3.79 (m, 1H), 2.78 (m, 2H), 2.70 (m, 1H), 2.27 (s, 3H).
MS (ESI-) m/z: 478.4 (M-H)⁻.

### EXAMPLE 42 : 4-acetyl-5-(2-chloro-pyridin-3-yl)-3-hydroxy-1-phenethyl-1,5-dihydro-pyrrol-2-one

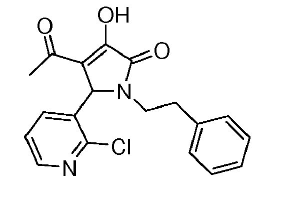

### Prepared using procedure I using :

2-chloro-3-pyridinecarboxaldehyde, 2-phenethylamine and ethyl acetopyruvate 14%, pink powder.
¹H NMR (300 MHz, DMSO-d₆): δ 8.33 (m, 1H), 7.39 - 7.09 (m, 7H), 5.48 (bs, 1H), 3.75 (m, 1H), 2.77 (m, 2H), 2.64 (m, 1H), 2.24 (bs, 3H).
MS (ESI-) m/z: 357.0 (M+H)⁺.

### EXAMPLE 43 : 4-acetyl-3-hydroxy-1-phenethyl-5-(6-trifluoromethyl-pyridin-3-yl)-1,5-dihydro-pyrrol-2-one

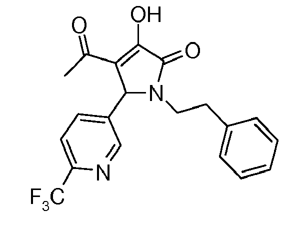

### Prepared using procedure I using :

6-trifluoromethyl-3-pyridinecarboxaldehyde, 2-phenethylamine and ethyl acetopyruvate 10%, white powder.
¹H NMR (300 MHz, DMSO-d₆): δ 8.64 (s, 1H), 7.79 (bs, 2H), 7.25 - 7.21 (m, 3H), 7.11 (m, 2H), 5.25 (bs, 1H), 3.82 (m, 1H), 2.83 (m, 2H), 2.76 (m, 1H), 2.27 (s, 3H). MS (ESI-) m/z: 391.0 (M+H)⁺.

### EXAMPLE 44 : 4-Acetyl-5-(4-bromo-phenyl)-1-[2-(2-chloro-phenyl)-ethyl]-3-hydroxy-1,5-dihydro-pyrrol-2-one

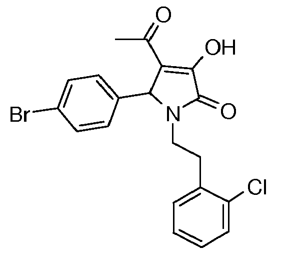

### Prepared using procedure I using :

4-bromobenzaldehyde, 2-chlorophenethylamine and ethyl-2,4-dioxovalerate 49%, white powder.
¹H NMR (300 MHz, CDCl₃-d): δ 7.47 (AB system, 2H, *J*=8.4 Hz), 7.34 (m, 1H), 7.18 (m, 3H), 6.95 (AB system, 2H, J=8.4 Hz), 4.81 (s, 1H), 3.90 (m, 1H), 3.00 (m, 3H), 2.02 (s, 3H).
MS (ESI+) m/z: 433.9 (M+H)⁺.

### EXAMPLE 45: 4-Acetyl-5-(4-chlorophenyl)-1-[2-(4-bromophenyl)-ethyl]-3-hydroxy-1,5-dihydro-pyrrol-2-one

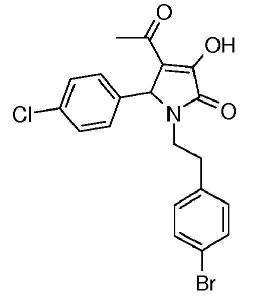

### Prepared using procedure I using :

4-chlorobenzaldehyde, 4-bromophenethylamine and ethyl-2,4-dioxovalerate 50%, white powder.
¹H NMR (300 MHz, CDCl₃-d): δ 7.40 (AB system, 2H, *J*=8.3 Hz), 7.34 (AB system, 2H, *J*=8.4 Hz), 7.02 (AB system, 2H, *J*=8.3 Hz), 6.96 (AB system, 2H, *J*=8.3 Hz), 4.85 (s, 1H), 3.91 (m, 1H), 3.00 - 2.65 (m, 3H), 2.05 (s, 3H).
MS (ESI+) m/z: 434.1 (M+H)⁺.

### EXAMPLE 46 : 4-Acetyl-5-(4-chlorophenyl)-1-[2-(4-chlorophenyl)-ethyl]-3-hydroxy-1,5-dihydro-pyrrol-2-one

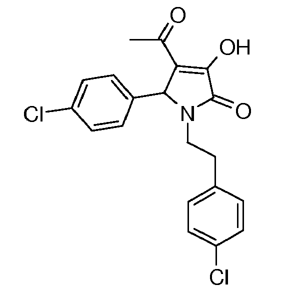

### Prepared using procedure I using :

4-chlorobenzaldehyde, 4-chlorophenethylamine and ethyl-2,4-dioxovalerate 42%, white powder.
¹H NMR (300 MHz, CDCl₃-d): δ 7.34 (AB system, 2H, *J*=8.4 Hz), 7.25 (m, 2H), 7.02 (AB system, 4H, *J*=8.3 Hz), 4.85 (s, 1H), 3.90 (m, 1H), 3.00 - 2.65 (m, 3H), 2.06 (s, 3H).
MS (ESI+) m/z: 389.9 (M+H)⁺.

### EXAMPLE 47 : 4-Acetyl-5-(4-bromophenyl)-1-[2-(2,4-dichlorophenyl)-ethyl]-3-hydroxy-1,5-dihydro-pyrrol-2-one

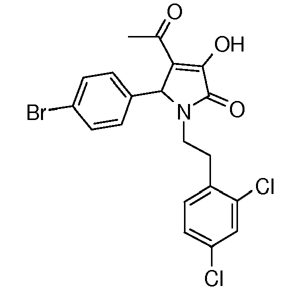

### Prepared using procedure I using :

4-bromobenzaldehyde, 2,4- dichlorophenethylamine and ethyl-2,4-dioxovalerate 60%, white powder.
¹H NMR (300 MHz, CDCl₃-d): δ 7.48 (AB system, 2H, *J*=8.3 Hz), 7.33 (d, 1H, *J*=1.8 Hz), 7.17 (dd, 1H, *J*=8.2 Hz, *J*=1.9 Hz), 7.10 (AB system, 1H, *J*=8.2 Hz), 6.99 (d, 2H, *J*=8.3 Hz), 4.91 (s, 1H), 3.83 (m, 1H), 3.10 - 2.80 (m, 3H), 2.05 (s, 3H).
MS (ESI+) m/z: 469.9 (M+H)⁺.

### EXAMPLE 48 : 4-acetyl-5-(4-bromo-3-chloro-phenyl)-3-hydroxy-1-phenethyl-1,5-dihydro-pyrrol-2-one

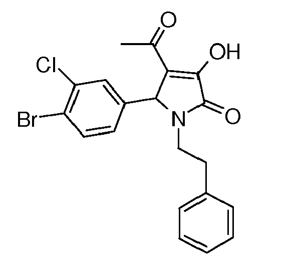

### Prepared using procedure I using :

4-bromo-3-chlorobenzaldehyde (Preparation C), 2-phenethylamine and ethyl-2,4-dioxovalerate 16%, white powder.
¹H NMR (300 MHz, DMSO-d₆): δ 7.68 (d, 1H, *J*=8.2 Hz), 7.43 (s, 1H), 7.22 (m,
3H), 7.12 (m, 2H), 7.01 (d, 1H, *J*=7.6 Hz), 6.94 (AB system, 2H, *J*=8.2 Hz), 5.09 (s, 1H), 3.81 (m, 1H), 2.79 (m, 2H), 2.71 (m, 1H), 2.27 (s, 3H).
MS (ESI+) m/z: 436.1 (M+H)⁺.

### EXAMPLE 49 : 4-acetyl-3-hydroxy-5-(3-hydroxy-4-methoxy-phenyl)-1-phenethyl-1,5-dihydro-pyrrol-2-one

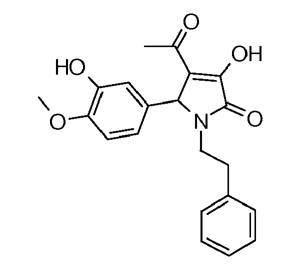

### Prepared using procedure I using :

3-hydroxy-4-methoxybenzaldehyde, 2-phenethylamine and ethyl-2,4-dioxovalerate 30%, pink powder.
¹H NMR (300 MHz, DMSO-d₆): δ 8.96 (s, 1H), 7.25 (m, 2H), 7.20 (AB system, 1H, *J*=6.9 Hz), 7.11 (AB system, 2H, *J*=6.9 Hz), 6.85 (AB system, 1H, *J*=8.2 Hz), 6.60 (AB system, 2H, *J*=7.5 Hz), 6.47 (s, 1H), 4.91 (s, 1 H), 3.73 (bs, 4H), 2.75 (m, 2H), 2.61 (m, 1H), 2.24 (s, 3H).
MS (ESI+) m/z: 368.2 (M+H)⁺.

### EXAMPLE 50 : 4-acetyl-1-(2-benzo[1,3]dioxol-5-yl-ethyl)-5-(4-chloro-phenyl)-3-hydroxy-1,5-dihydro-pyrrol-2-one

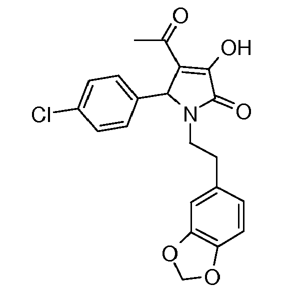

### Prepared using procedure I using :

4-chlorobenzaldehyde, 2-Benzo[1,3]dioxol-5-yl-ethylamine (commercially available) and ethyl-2,4-dioxovalerate 29%, white powder.
¹H NMR (300 MHz, DMSO-d₆): δ 7.37 (AB system, 1H, *J*=8.3 Hz), 7.18 (AB system, 2H, *J*=8.3 Hz), 6.78 (AB system, 1H, *J*=7.8 Hz), 6.68 (s, 1H), 6.56 (AB system, 1H, *J*=7.2 Hz), 5.95 (d, 2H, *J_{gem}*=2.3 Hz), 5.11 (s, 1H), 3.73 (m, 1H), 2.68 (m, 2H), 2.60 (m, 1H), 2.26 (s, 3H).
MS (ESI+) m/z: 400.1 (M+H)⁺.

### EXAMPLE 51 : 4-acetyl-5-(4-bromo-phenyl)-3-hydroxy-1-[2-(3-trifluoromethylphenyl)-ethyl]-1,5-dihydro-pyrrol-2-one

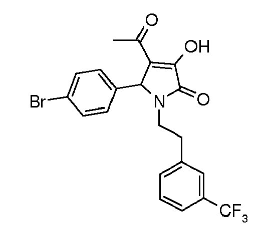

### Prepared using procedure I using :

4-bromobenzaldehyde, 3-(trifluoromethyl)phenethylamine and ethyl-2,4-dioxovalerate 41 %, white powder.
¹H NMR (300 MHz, CDCl₃-d): δ 7.49 (m, 3H), 7.41 (t, 1H, *J*=7.6 Hz), 7.32 (m, 2H), 6.94 (AB system, 2H, *J*=8.2 Hz), 4.79 (s, 1H), 3.92 (m, 1H), 3.05 - 2.73 (m, 3H), 2.05 (s, 3H).
MS (ESI+) m/z: 468.2 (M+H)⁺.

### EXAMPLE 52 : 4-acetyl-5-(4-bromo-phenyl)-3-hydroxy-1-indan-2-yl-1,5-dihydro-pyrrol-2-one

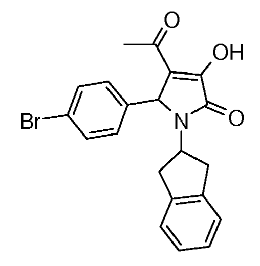

### Prepared using procedure I using :

4-bromobenzaldehyde, 2-aminoindane and ethyl-2,4-dioxovalerate 34%, pale pink powder.
¹H NMR (300 MHz, CDCl₃-d): δ 7.39 (AB system, 2H, *J*=8.4 Hz), 7.11 (m, 3H), 6.95 (m, 3H), 5.12 (s, 1H), 4.56 (quint., 1H, *J*= 7.7 Hz), 3.30 (dd, 1H, *J*= 7.3 Hz, 15.8 Hz), 2.96 (dd, 3H, *J*= 7.8 Hz, 15.8 Hz), 2.11 (s, 3H).
MS (ESI+) m/z: 412.1 (M+H)⁺.

### EXAMPLE 53 : 4-acetyl-5-(4-bromophenyl)-1-[2-(3-bromophenyl)-ethyl]-3-hydroxy-1,5-dihydro-pyrrol-2-one

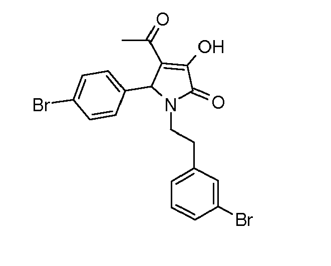

### Prepared using procedure I using :

4-bromobenzaldehyde, 3- bromophenethylamine and ethyl-2,4-dioxovalerate 21 %, white crystals.
¹H NMR (300 MHz, CDCl₃-d): δ 7.50 (AB system, 2H, *J*=8.4 Hz), 7.39 (AB system, 1H, *J*=8.6 Hz), 7.24 (s, 1H), 7.16 (t, 1H, *J*=7.7 Hz), 7.02 (d, 1H, *J*=7.6 Hz), 6.94 (AB system, 2H, *J*=8.4 Hz), 4.77 (s, 1H), 3.91 (m, 1H), 3.00 - 2.68 (m, 3H), 2.02 (s, 3H).
¹³C NMR (75.5 MHz, CDCl₃-d): δ 194.23 (C), 164.74 (C), 157.94 (C), 140.45 (C), 133.79 (C), 132.56 (CH), 131.84 (CH), 130.46 (CH), 130.17 (CH), 129.53 (CH), 127.47 (CH), 123.41 (C), 122.91 (C), 119.89 (C), 61.38 (CH), 42.15 (CH₂), 34.19 (CH₂), 28.57 (CH₃).

### EXAMPLE 54 : 4-acetyl-5-(4-bromophenyl)-3-hydroxy-1-(2-m-tolyl-ethyl)-1,5-dihydro-pyrrol-2-one

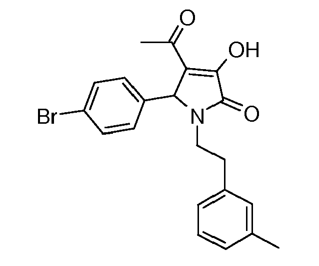

### Prepared using procedure I using :

4-bromobenzaldehyde, 3- methylphenethylamine and ethyl-2,4-dioxovalerate 8%, white powder.
¹H NMR (300 MHz, CDCl₃-d): δ 7.48 (AB system, 2H, *J*=8.3 Hz), 7.18 (t, 1H, *J*=7.4 Hz), 7.06 (AB system, 1H, *J*=7.4 Hz), 6.90 (m, 4H), 4.74 (s, 1H), 3.95 (m, 1H), 2.92 - 2.65 (m, 3H), 2.32 (s, 3H), 2.01 (s, 3H).
MS (ESI+) m/z: 416.1 (M+H)⁺.

### EXAMPLE 55 : 4-Acetyl-5-(4-bromo-phenyl)-3-hydroxy-1-[2-(4-morpholin-4-ylphenyl)-ethyl]-1,5-dihydro-pyrrol-2-one

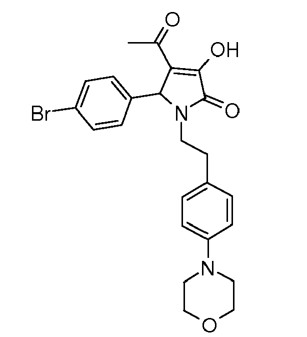

### Prepared using procedure I using :

4-bromobenzaldehyde, 2-(4-morpholin-4-yl-phenyl)-ethylamine (synthesized according to preparation D, E and F), and Ethyl-2,4-dioxovalerate 29%, pale brown powder.
¹H NMR (300 MHz, CDCl₃-d): δ 7.48 (m, 2H), 6.97 (m, 4H), 6.84 (m, 2H), 4.82 (2s, 1H), 3.94 (m, 1H), 3.86 (m, 4H), 3.13 (m, 4H), 2.92 - 2.61 (m, 3H), 2.04 (2s, 3H).
MS (ESI+) m/z: 485.2 (M+H)⁺.

### EXAMPLE 56: 4-acetyl-5-(4-bromophenyl)-3-hydroxy-1-(2-naphthalen-1-yl-ethyl)-1,5-dihydro-pyrrol-2-one

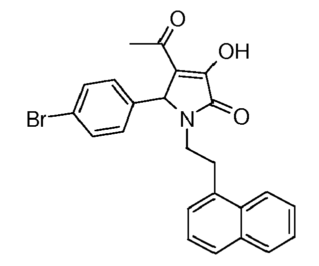

### Prepared using procedure I using :

4-bromobenzaldehyde, 2-(1-naphthyl)ethylamine hydrochloride and ethyl-2,4-dioxovalerate
34%, white powder.
¹H NMR (300 MHz, CDCl₃-d): δ 7.88 (AB system, 1H, *J*=7.5 Hz), 7.77 (m, 2H), 7.50 (m, 2H), 7.36 (m, 3H), 6.64 (d, 2H, *J*=8.3 Hz), 4.41 (s, 1H), 3.98 (m, 1H), 3.45 - 3.19 (m, 2H), 3.10 (m, 1H), 1.91 (s, 3H).
MS (ESI+) m/z: 450.2 (M+H)⁺.

### Representative cysteine proteases

### USP7 Protein production & purification

The cDNA encoding USP7 was obtained by PCR amplification from placenta mRNA. USP7 cDNA was subcloned by PCR into a baculovirus expression vector (pFastBac-HT; Invitrogen). Full-length wild-type human USP7 and its catalytic mutant (cysteine 223 replaced by alanine, C223A) were produced as N-terminally His-tagged fusions in *Spodoptera frugiperda* cells (Sf9, Invitrogen), using the Bac-to-Bac Baculovirus system from Invitrogen according to the manufacturer's instructions. pFastBac-HT-B-USP7 was used to transform DH10bac cells (Invitrogen), and blue/white selection was carried out on X-gal/IPTG agar plates. Bacmid DNA was prepared by an alkaline lysis procedure. The integrity of the bacmid minipreps and their orientation were checked by PCR, using generic and specific primers. Sf9 cells were cultured in InsectXpress medium (Cambrex) at 27°C and transfected with the corresponding bacmid, using GeneShuttle 40 (Q-BIOgen). Viruses were recovered in the supernatant 72 h after transfection. Viruses were amplified by infecting Sf9 cells in 50 ml InsectXpress medium in a 150 cm² cell culture flask with 500 µl of the supernatant from transfected Sf9 cells. Following the second round of amplification, Sf9 cells were recovered by rapid SDS lysis, boiled for 5 min at 100°C, sonicated briefly and centrifuged for 20 min at 14,000 g. Expression levels in infected Sf9 cells were compared with those in uninfected cells. Fusion proteins were then allowed to bind to TALON beads (BD Biosciences, TALON metal affinity resin) for 30 min at 4°C with gentle rocking. Beads were extensively washed (50 mM sodium phosphate buffer pH 7.0, 500 mM NaCl, 10 mM Imidazole, 0.5% Triton X-100 and 10% glycerol) and bound proteins were eluted in wash buffer supplemented with 250 mM Imidazole (Sigma). Eluted fractions were resolved on 4-12% NuPAGE gels (Novex, Invitrogen). Fractions containing high concentrations of purified proteins (purity > 95%) were dialyzed (20 mM Tris HCl pH 7.6, 200 mM NaCl, 1 mM DTT, 1 mM EDTA and 10% glycerol) were aliquoted and snap frozen in liquid nitrogen before storage at -80°C.

### USP7 activity assay

USP7 was diluted in USP buffer (50 mM Tris HCl; 0.5 mM EDTA; 5 mM DTT; 0.01 % Triton X-100; Bovine Serum Albumin 0.05 mg.ml⁻¹ pH7.6). Compounds stocks (100 mM) were stored at -20°C in DMSO. Compounds were tested at different concentrations: from 200 µM to 91 nM.

Reactions were performed as duplicates in Black LJL 96 well plates (HE microplates; Molecular Devices; 20 µl final reaction volume). The substrate concentration for USP7 was 300 nM Ub-AMC (Chem. Biol., 2003, 10, p. 837-846) (Boston Biochem). The concentrations of the enzyme (USP7) in specificity assays was 100 pM. The concentrations were determined in order to perform specificity assays under initial velocities at fixed substrate concentration. Compounds were pre-incubated with enzymes for 30 minutes at 25°C. Reactions were initiated by addition of substrate to the plates containing the enzymes (+/- compounds) diluted in assay buffer. Reactions were incubated for 60 minutes at 37°C. Reactions were stopped by adding acetic acid (100 mM final). Readings were performed on a Pherastar Fluorescent Reader (BMG). λ Emission 380 nm; λ Excitation = 460 nm. Data (mean values +/- standard deviation) were analyzed as % of control (no compound) and plotted as percentage versus the Log of the compound concentration using GraphPad (Prism). Data were fitted to a sigmoidal model (variable slope).

### USP5 activity assay

USP5 was diluted in USP buffer (50 mM Tris HCl; 0.5 mM EDTA; 5 mM DTT; 0.01% Triton X-100; Bovine Serum Albumin 0.05 mg.ml⁻¹ pH 7.6). Compounds stocks (100 mM) were stored at -20°C in DMSO. Compounds were tested at different concentrations: from 200 µM to 91 nM.

Reactions were performed as duplicates in Black LJL 96 well plates (HE microplates; Molecular Devices; 20 µl final reaction volume). The substrate concentration for USP5 was 300 nM Ub-AMC (Boston Biochem). The concentrations of the enzyme (USP5) in specificity assays was 300 pM. The concentrations were determined in order to perform specificity assays under initial velocities at fixed substrate concentration. Compounds were pre-incubated with enzymes for 30 minutes at 25°C. Reactions were initiated by addition of substrate to the plates containing the enzymes (+/- compounds) diluted in assay buffer. Reactions were incubated for 60 minutes at 37°C. Reactions were stopped by adding acetic acid (100 mM final). Readings were performed on a Pherastar Fluorescent Reader (BMG). λ Emission 380 nm; λ Excitation = 460 nm. Data (mean values +/- standard deviation) were analyzed as % of control (no compound) and plotted as percentage versus the Log of the compound concentration using GraphPad (Prism). Data were fitted to a sigmoidal model (variable slope).

### Cloning & purification of USP8

The cDNA encoding USP8 was obtained by PCR amplification from placenta mRNA. USP8 cDNA was subcloned by PCR into a baculovirus expression vector (pFastBac-HT; Invitrogen). A cDNA encoding a mutated USP8 was generated by mutagenic PCR. The corresponding protein encodes a cysteine to alanine substitution at residue 786. The sequences were ascertained by sequencing of the entire open reading frame. Bacmids encoding USP7 were generated following DH10bac transposition. The corresponding bacmids were transfected into insect cells (Sf9). Viruses were recovered from culture supernatant and amplified twice. Insect cells (Sf9 or High Five; Invitrogen) were infected for 72 hours. Total cell lysates were harvested and lyzed in lysis buffer (Tris HCl 50 mM pH7.6; 0.75 % NP40; 500 mM NaCl; 10 % glycerol; 1 mM DTT; 10 mM imidazole; Protease Inhibitor Cocktail; AEBSF 20 µg.ml⁻¹; Aprotinin 10 µg.ml⁻¹). Proteins were affinity purified on metal affinity resins (Talon Metal affinity resin; BD Biosciences). Bound materials were extensively washed in wash buffer (50 mM Sodium Phosphate pH 7.0; 300 mM NaCl; 10 mM imidazole; 0.5% Triton X-1 00; 10% glycerol) and eluted from the resin in 250 mM imidazole-containing wash buffer. Proteins were dialyzed in dialysis buffer (Tris HCl pH 7.6 20 mM; NaCl 200 mM; DTT 1 mM; EDTA 1 mM; 10% Glycerol). Proteins purifications were analyzed on 4-12% NuPAGE (Invitrogen).

### USP8 activity assay

USP8 was diluted in USP buffer (50 mM Tris HCl; 0.5 mM EDTA; 5 mM DTT; 0.01% Triton X-100; Bovine Serum Albumin 0.05 mg.ml⁻¹ pH8.8). Compounds stocks (100 mM) were stored at -20°C in DMSO. Compounds were tested at different concentrations: from 200 µM to 91 nM.

Reactions were performed as duplicates in Black LJL 96 well plates (HE microplates; Molecular Devices; 20 µl final reaction volume). The substrate concentration for USP8 was 400 nM Ub-AMC (Boston Biochem). The concentration of the enzyme (USP8) in specificity assays was 1.36 nM. The concentrations were determined in order to perform specificity assays under initial velocities at fixed substrate concentration. Compounds were pre-incubated with enzymes for 30 minutes at 25°C. Reactions were initiated by addition of substrate to the plates containing the enzymes (+/- compounds) diluted in assay buffer. Reactions were incubated for 60 minutes at 37°C. Reactions were stopped by adding acetic acid (100 mM final). Readings were performed on a Pherastar Fluorescent Reader (BMG). λ Emission 380 nm : λ Excitation = 460 nm. Data (mean values +/- standard deviation) were analyzed as % of control (no compound) and plotted as percentage versus the Log of the compound concentration using GraphPad (Prism). Data were fitted to a sigmoidal model (variable slope).

### UCH-L1 activity assay

UCH-L1 was diluted in USP buffer (50 mM Tris HCl; 0.5 mM EDTA; 5 mM DTT; 0.01% Triton X-100; Bovine Serum Albumin 0.05 mg.ml⁻¹ pH7.6). Compounds stocks (100 mM) were stored at -20°C in DMSO. Compounds were tested at different concentrations: from 200 µM to 91 nM.

Reactions were performed as duplicates in Black LJL 96 well plates (HE microplates; Molecular Devices; 20 µl final reaction volume). The substrate concentration for UCH-L1 was 400 nM Ub-AMC (Boston Biochem). The concentration of the enzyme (UCH-L1) in specificity assays was 2.5 nM. The concentrations were determined in order to perform specificity assays under initial velocities at fixed substrate concentration. Compounds were pre-incubated with enzymes for 30 minutes at 25°C. Reactions were initiated by addition of substrate to the plates containing the enzymes (+/- compounds) diluted in assay buffer. Reactions were incubated for 60 minutes at 37°C. Reactions were stopped by adding acetic acid (100 mM final). Readings were performed on a Pherastar Fluorescent Reader (BMG). λ Emission 380 nm; λ Excitation = 460 nm. Data (mean values +/- standard deviation) were analyzed as % of control (no compound) and plotted as percentage versus the Log of the compound concentration using GraphPad (Prism). Data were fitted to a sigmoidal model (variable slope).

### UCH-L3 activity assay

UCH-L3 was diluted in USP buffer (50 mM Tris HCl; 0.5 mM EDTA; 5 mM DTT; 0.01% Triton X-100; Bovine Serum Albumin 0.05 mg.ml⁻¹ pH7.6). Compounds stocks (100 mM) were stored at -20°C in DMSO. Compounds were tested at different concentrations: from 200 µM to 91 nM.

Reactions were performed as duplicates in Black LJL 96 well plates (HE microplates; Molecular Devices; 20 µl final reaction volume). The substrate concentration for UCH-L3 was 400 nM Ub-AMC (Boston Biochem). The concentration of the enzyme (UCH-L3) in specificity assays was 13 pM. The concentrations were determined in order to perform specificity assays under initial velocities at fixed substrate concentration. Compounds were pre-incubated with enzymes for 30 minutes at 25°C. Reactions were initiated by addition of substrate to the plates containing the enzymes (+/- compounds) diluted in assay buffer. Reactions were incubated for 60 minutes at 37°C. Reactions were stopped by adding acetic acid (100 mM final). Readings were performed on a Pherastar Fluorescent Reader (BMG). λ Emission 380 nm; λ Excitation = 460 nm. Data (mean values +/- standard deviation) were analyzed as % of control (no compound) and plotted as percentage versus the Log of the compound concentration using GraphPad (Prism). Data were fitted to a sigmoidal model (variable slope).

### Caspase 3 activity assay

Caspase 3 was diluted in Caspase 3 buffer (100 mM Hepes pH 7.5; 10% sucrose; 0.1% CHAPS). Compounds stocks (100 mM) were stored at -20°C in DMSO. Compounds were tested at different concentrations: from 200 µM to 91 nM.

Reactions were performed as duplicates in Black LJL 96 well plates (HE microplates; Molecular Devices; 20 µl final reaction volume). The substrate concentration for caspase 3 specificity assay was 250 nM (Ac-DEVD-AMC; Promega). The concentration of the enzyme (Caspase 3) in specificity assays was 1.6 nM. The concentrations were determined in order to perform specificity assays under initial velocities at fixed substrate concentration. Compounds were pre-incubated with enzymes for 30 minutes at 25°C. Reactions were initiated by addition of substrate to the plates containing the enzymes (+/- compounds) diluted in assay buffer. Reactions were incubated for 60 minutes at 37°C. Reactions were stopped by adding acetic acid (100 mM final). Readings were performed on a Pherastar Fluorescent Reader (BMG). λ Emission 380 nm; λ Excitation = 460 nm. Data (mean values +/- standard deviation) were analyzed as % of control (no compound) and plotted as percentage versus the Log of the compound concentration using GraphPad (Prism). Data were fitted to a sigmoidal model (variable slope).

### Cell viability and proliferation methods

### HCT116 cell viability and proliferation assay

HCT116 colon cancer cells were obtained from ATCC (American Type Culture Collection), and maintained in Mc Coy's 5A medium containing 10% FBS, 3 mM glutamine and 1% penicillin/streptomycin. Cells were incubated at 37°C in a humidified atmosphere containing 5% CO₂.

Cell viability was assayed using the MTS technique in 96-well culture plates (CellTiter 96^{®} Aqueous Non-Radioactive Cell Proliferation Assay, Promega) according to the manufacturer's instructions. MTS (3-(4,5-dimethyl-thiazol-2-yl)-5-(3-carboxymethoxyphenyl)-2-(4-sulfophenyl)-2H-tetra-zolium) is a MTT-derived tetrazolium that is reduced in metabolically active cells into a soluble, cell-permeant formazan. The amount of formazan, detected by its absorbance at 492 nm is proportional to the number of living, metabolically active cells.

10³ HCT116 cells were seeded per well. 24 hours later, the medium was changed and the cells treated in triplicate with the concentrations of each compound from 250 to 2 µM. The compounds were diluted in 100% DMSO, whose final concentration on cells was kept at 0.5%.

Cells were incubated with the compounds for 72 hours, and their viability then assayed by the addition of MTS for 2 hours. Absorbance at 492 nm was measured directly from the 96-well culture plates. G150 (Growth Inhibition 50) concentrations for each compound were calculated using a sigmoidal variable slope fit (Prism 4.0, Graphpad Softwares). Values represent mean of three independent experiments.

### RESULTS

### 1. Inhibition of cysteine protease activities

**USPs**

| **Examples** | **MW** | **MLogP** | **USP7** | **USP8** | **USP 5** | **Uch-L1** | **Uch-L3** | **Casp3** |
|---|---|---|---|---|---|---|---|---|
| **25** | 434, 72 | 3,2 | 3,10 | **>200** | >200 | >200 | >200 | >200 |
| **26** | 479,18 | 3,3 | 2,70 | **>200** | >200 | >200 | >200 | >200 |
| **28** | 469,17 | 3,7 | 1,61 | **>200** | >200 | >200 | >200 | >200 |
| **30** | 430,30 | 2,4 | 11,15 | **>200** | >200 | >200 | >200 | >200 |
| **32** | 430,30 | 2,4 | 5,28 | **>200** | >200 | >200 | >200 | >200 |
| **33** | 418,27 | 3,1 | 3,05 | **>200** | >200 | >200 | >200 | >200 |
| **34** | 444, 33 | 2,6 | 6,60 | **>200** | >200 | >200 | >200 | >200 |
| **37** | 365,38 | 2,1 | 7,03 | **>200** | >200 | >200 | >200 | >200 |
| **41** | 479,18 | 3,3 | 8,98 | **>200** | >200 | >200 | >200 | >200 |
| **42** | 356,81 | 1,6 | 12,00 | **>200** | >200 | >200 | >200 | >200 |
| **45** | 434, 72 | 3,2 | 4,20 | **>200** | >200 | >200 | >200 | >200 |
| **46** | 390,27 | 3,1 | 4,40 | **>200** | >200 | >200 | >200 | >200 |
| **47** | 469,17 | 3,7 | 1,90 | **>200** | >200 | >200 | >200 | >200 |

## Claims

1. A compound of formula (I) 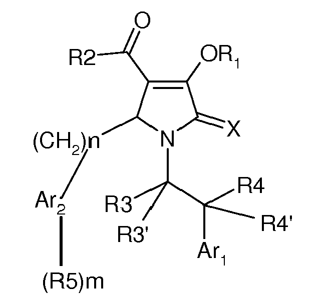 wherein :
Ar1 represents an aryl or heteroaryl group, said aryl or heteroaryl being optionally substituted by one or more substituent(s) chosen from : halogen atom, -alkyl, -aryl, -heterocycle, -alkylaryl, -CN, -NO₂, perfluoroalkyl-, perfluoroalkoxy-, polyfluoroalkyl-, polyfluoroalkoxy-, -OR, -NRR', =O, -C(=O)R, -C(=O)NRR', -C(=O)OR, -S(O)pR, -OC(=O)R; and/or said aryl or heteroaryl being optionally fused with an heterocyclic ring;
Ar2 represents an aryl or heteroaryl group, said aryl or heteroaryl,
n is an integer chosen from 0 to 6;
XisaOorSatom;
R1 represents a hydrogen atom or an -alkyl group;
R2 represents an alkyl, alkenyl, -alkylaryl, -alkylheteroaryl, -alkenylaryl, -Oalkyl, -NRR', cycloalkyl, aryl or heteroaryl group, said cycloalkyl, aryl or heteroaryl being optionally fused with a saturated or unsaturated cycloalkyl or heterocyclic ring and/or being optionally substituted by one or more substituent(s) chosen from : halogen atom, -alkyl;
R3, R3', R4, R4' identical or different independently represent a hydrogen atom or an -alkyl, -aryl, -alkylaryl; and/or
wherein one of R3 or R3' forms a bond with an ortho-atom of Ar1 so as to form a cycloalkyl or heterocycle fused wit Ar1 ;
Each R5 independently represents a halogen atom, -alkyl, -aryl, -alkylaryl, -CN, -NO₂, perfluoroalkyl-, perfluoroalkoxy-, polyfluoroalkyl-, polyfluoroalkoxy-, -OR, -NRR', =O, -C(=O)R, -C(=O)NRR', -C(=O)OR, -NRC(=O)R', -S(O)pR, -OC(=O)R, heteroaryl, heterocyclic ring, -O-alkyl-NRR',
and/or two R5 form with Ar2 to which they are attached a saturated or unsaturated cycloalkyl or heterocyclic ring, fused to Ar2,
and/or one R5 and R3 or R3' form a bond, so as to form a N-containing heterocyclic ring, and generate a tricyclic fused structure, comprising the pyrrolone, said form N-containing heterocycle and Ar2;
where R, R' identical or different, independently represent a hydrogen atom or an -alkyl, -aryl, -alkylaryl;
m is 0, or an integer chosen from 1 to 5;
p is 0, 1 or 2;
as well as its tautomers and its pharmaceutically acceptable salts.

2. The compound according to claim 1, wherein Ar1 represents an aryl, preferably phenyl or naphtyl, or heteroaryl group, preferably thienyl, pyridyl, said aryl or heteroaryl being optionally substituted by one or more substituent(s) chosen from halogen atom, -aryl, such as phenyl, -OR, heterocycle, such as morpholinyl; and/or said aryl or heteroaryl being optionally fused with an heterocyclic ring, such as an oxiran.

3. The compound according to claim 1 or 2, wherein R3=R3'=R4=R4'=H; and/or wherein one of R3 or R3' forms a bond with an ortho-atom of Ar1 so as to form a cycloalkyl, such as cylcopentyl, fused wit Ar1.

4. The compound according to claim 1, 2 or 3, wherein Ar2 represents an aryl, preferably phenyl, or heteroaryl group, preferably pyridyl, said aryl or heteroaryl being optionally substituted by one or more substituent(s) chosen from : halogen atom, -alkyl, -aryl, -CN, -NO₂, perfluoroalkyl-, perfluoroalkoxy-, polyfluoroalkoxy-, -OR, -C(=O)OR, -NRC(=O)R', -S(O)pR, -OC(=O)R, heteroaryl, such as tetrazolyl, heterocyclic, preferably morpholinyl, ring, -O-alkyl-NRR'; and/or said aryl or heteroaryl being optionally fused with a saturated heterocyclic ring such as dioxolanyl;

5. The compound according to anyone of the preceding claims, wherein Ar2 is a phenyl group substituted on the para position.

6. The compound according to anyone of the preceding claims wherein n is 0.

7. The compound according to anyone of the preceding claims wherein X is a S atom.

8. The compound according to anyone of the preceding claims, wherein R1 represents a hydrogen atom or an -alkyl group, more preferably a hydrogen atom.

9. The compound according to anyone of the preceding claims, wherein R2 represents an alkyl, -alkenylaryl, -Oalkyl, cycloalkyl, aryl, such as phenyl, or heteroaryl group, such as pyridyl, said cycloalkyl, aryl or heteroaryl being optionally fused with a saturated heterocyclic ring such as dioxolanyl and/or being optionally substituted by one or more substituent(s) chosen from : halogen atom, -alkyl; more preferably R2 represents a -CHRR' group.

10. The compound according to anyone of the preceding claims chosen from the group consisting in:
4-acetyl-5-(3,4-dichloro-phenyl)-3-hydroxy-1-phenethyl-1,5-dihydro-pyrrol-2-one
4-acetyl-3-hydroxy-1-phenethyl-5-(4-cyanophenyl)-1,5-dihydro-2H-pyrrol-2-one
4-Acetyl-5-(4-bromo-phenyl)-3-hydroxy-1-phenethyl-1,5-dihydro-pyrrol-2-one
4-acetyl-3-hydroxy-1-phenethyl-5-(4-acetoxyphenyl)-1,5-dihydro-2H-pyrrol-2-one
4-acetyl-3-hydroxy-1-phenethyl-5-(4-trifluoromethoxyphenyl)-1,5-dihydro-2H-pyrrol-2-one
4-acetyl-3-hydroxy-1-phenethyl-5-(4-difluoromethoxyphenyl)-1,5-dihydro-2H-pyrrol-2-one
4-acetyl-3-hydroxy-1-phenethyl-5-(4-butoxyphenyl)-1,5-dihydro-2H-pyrrol-2-one
4-Acetyl-3-hydroxy-1-phenethyl-5-(4-trifluoromethyl-phenyl)-1,5-dihydro-pyrrol-2-one
4-acetyl-3-hydroxy-1-phenethyl-5-(4-n-octyloxyphenyl)-1,5-dihydro-2H-pyrrol-2-one
4-acetyl-5-[4-(3-di methylam i nopropoxy)-phenyl]-3-hydroxy-1-phenethyl-1,5-dihydro-2H-pyrrol-2-one
4-acetyl-3-hydroxy-1-phenethyl-5-(4-morpholinylphenyl)-1,5-dihydro-2H-pyrrol-2-one
4-acetyl-5-(4-bromo-phenyl)-3-hydroxy-1-(2-pyridin-4-yl-ethyl)-1,5-dihydro-pyrrol-2-one
4-acetyl-3-hydroxy-1-phenethyl-5-[4-(1H-tetrazol-5-yl)-phenyl]-1,5-dihydro-2H-pyrrol-2-one
4-acetyl-3-hydroxy-5-(4-isopropoxyphenyl)-1-phenethyl-1,5-dihydro-2H-pyrrol-2-one
4-acetyl-5-(4-bromo-phenyl)-3-hydroxy-1-(2-thiophen-2-yl-ethyl)-1,5-dihydro-pyrrol-2-one
4-acetyl-5-(4-bromo-2-fluoro-phenyl)-3-hydroxy-1-phenethyl-1,5-dihydro-2H-pyrrol-2-one
4-acetyl-1-(2-biphenyl-4-yl-ethyl)-5-(4-bromo-phenyl)-3-hydroxy-1,5-dihydro-pyrrol-2-one
4-acetyl-5-(4-bromo-phenyl)-3-hydroxy-1-(3-phenyl-propyl)-1,5-dihydro-pyrrol-2-one
4-acetyl-5-(4-bromo-phenyl)-3-hydroxy-1-(4-phenyl-butyl)-1,5-dihydro-pyrrol-2-one
4-(3-acetyl-4-hydroxy-5-oxo-1-phenethyl-2,5-dihydro-1H-pyrrol-2-yl)-benzoic acid
4-acetyl-3-hydroxy-5-(4-methanesulfonyl-phenyl)-1-phenethyl-1,5-dihydro-2H-pyrrol-2-one
4-acetyl-3-hydroxy-1-phenethyl-5-pyridin-2-yl-1,5-dihydro-2H-pyrrol-2-one
4-acetyl-3-hydroxy-1-phenethyl-5-(4-hydroxy-phenyl)-1,5-dihydro-2H-pyrrol-2-one
4-acetyl-5-(4-tert-butyl-phenyl)-3-hydroxy-1-phenethyl-1,5-dihydro-2H-pyrrol-2-one
4-acetyl-5-(4-bromo-phenyl)-1-[2-(4-chloro-phenyl)-ethyl]-3-hydroxy-1,5-dihydro-pyrrol-2-one
4-acetyl-5-(4-bromo-phenyl)-1-[2-(4-bromo-phenyl)-ethyl]-3-hydroxy-1,5-dihydro-pyrrol-2-one
4-acetyl-5-biphenyl-4-yl-3-hydroxy-1-phenethyl-1,5-dihydro-2H-pyrrol-2-one
4-acetyl-5-(4-bromo-phenyl)-1-[2-(3,4-dichloro-phenyl)-ethyl]-3-hydroxy-1,5-dihydro-pyrrol-2-one
5-(4-bromo-phenyl)-4-cyclopropanecarbonyl-3-hydroxy-1-phenethyl-1,5-dihydro-pyrrol-2-one
4-acetyl-5-(4-bromo-phenyl)-3-hydroxy-1-[2-(3-methoxy-phenyl)-ethyl]-1,5-dihydro-pyrrol-2-one
4-acetyl-5-(4-bromo-phenyl)-3-hydroxy-1-[2-(2-methoxy-phenyl)-ethyl]-1,5-dihydro-pyrrol-2-one
4-acetyl-5-(4-bromo-phenyl)-3-hydroxy-1-[2-(4-methoxy-phenyl)-ethyl]-1,5-dihydro-pyrrol-2-one
4-acetyl-5-(4-bromo-phenyl)-3-hydroxy-1-[2-(4-fluorophenyl)-ethyl]-1,5-dihydro-pyrrol-2-one
4-acetyl-5-(4-bromo-phenyl)-3-hydroxy-1-[2-(4-ethoxy-phenyl)-ethyl]-1,5-dihydro-pyrrol-2-one
4-acetyl-3-hydroxy-1-phenethyl-5-pyridin-4-yl-1,5-dihydro-pyrrol-2-one
N-[4-(3-acetyl-4-hydroxy-5-oxo-1-phenethyl-2,5-dihydro-1H-pyrrol-2-yl)-phenyl]-acetamide
4-acetyl-5-benzo[1,3]dioxol-5-yl-3-hydroxy-1-phenethyl-1,5-dihydro-pyrrol-2-one
4-acetyl-3-hydroxy-1-phenethyl-5-pyridin-3-yl-1,5-dihydro-pyrrol-2-one
4-acetyl-5-benzo[1,3]dioxol-5-yl-1-[2-(3,4-dimethoxy-phenyl)-ethyl]-3-hydroxy-1,5-dihydro-pyrrol-2-one
4-acetyl-5-(4-chloro-3-fluoro-phenyl)-3-hydroxy-1-phenethyl-1,5-dihydro-pyrrol-2-one
4-acetyl-5-(3,4-dibromo-phenyl)-3-hydroxy-1-phenethyl-1,5-dihydro-pyrrol-2-one
4-acetyl-5-(2-chloro-pyridin-3-yl)-3-hydroxy-1-phenethyl-1,5-dihydro-pyrrol-2-one
4-acetyl-3-hydroxy-1-phenethyl-5-(6-trifluoromethyl-pyridin-3-yl)-1,5-dihydro-pyrrol-2-one
4-Acetyl-5-(4-bromo-phenyl)-1-[2-(2-chloro-phenyl)-ethyl]-3-hydroxy-1,5-dihydro-pyrrol-2-one
4-Acetyl-5-(4-chlorophenyl)-1-[2-(4-bromophenyl)-ethyl]-3-hydroxy-1,5-dihydro-pyrrol-2-one
4-Acetyl-5-(4-chlorophenyl)-1-[2-(4-chlorophenyl)-ethyl]-3-hydroxy-1,5-dihydro-pyrrol-2-one
4-Acetyl-5-(4-bromophenyl)-1-[2-(2,4-dichlorophenyl)-ethyl]-3-hydroxy-1,5-dihydro-pyrrol-2-one
4-acetyl-5-(4-bromo-3-chloro-phenyl)-3-hydroxy-1-phenethyl-1,5-dihydro-pyrrol-2-one
4-acetyl-3-hydroxy-5-(3-hydroxy-4-methoxy-phenyl)-1-phenethyl-1,5-dihydro-pyrrol-2-one
4-acetyl-1-(2-benzo[1,3]dioxol-5-yl-ethyl)-5-(4-chloro-phenyl)-3-hydroxy-1,5-dihydro-pyrrol-2-one
4-acetyl-5-(4-bromo-phenyl)-3-hydroxy-1-[2-(3-trifluoromethyl-phenyl)-ethyl]-1,5-dihydro-pyrrol-2-one
4-acetyl-5-(4-bromo-phenyl)-3-hydroxy-1-indan-2-yl-1,5-dihydro-pyrrol-2-one
4-acetyl-5-(4-bromophenyl)-1-[2-(3-bromophenyl)-ethyl]-3-hydroxy-1,5-dihydro-pyrrol-2-one
4-acetyl-5-(4-bromophenyl)-3-hydroxy-1-(2-m-tolyl-ethyl)-1,5-dihydro-pyrrol-2-one
4-Acetyl-5-(4-bromo-phenyl)-3-hydroxy-1-[2-(4-morpholin-4-yl-phenyl)-ethyl]-1,5-dihydro-pyrrol-2-one
4-acetyl-5-(4-bromophenyl)-3-hydroxy-1-(2-naphthalen-1-yl-ethyl)-1,5-dihydro-pyrrol-2-one
as well as their tautomers and pharmaceutically acceptable salts.

11. Process of preparation of a compound according to anyone of the preceding claims comprising the step of reacting corresponding compounds of formula (II), (III) and (IV): 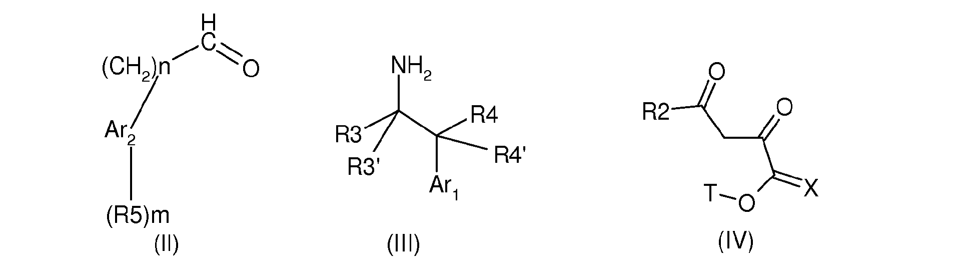 wherein R2, R3, R3', R4, R4', R5, Ar1, Ar2, X, m, n are defined as in any of the preceding claims and T represents an alkyl group optionally substituted by one or more substituent(s) chosen from: halogen atom, -alkyl, -aryl, -CN, -NO₂, perfluoroalkyl-.

12. A pharmaceutical composition comprising a compound of formula (I) as defined in anyone of claims 1 to 10 as well as its tautomers and pharmaceutically acceptable salts, and a pharmaceutical acceptable excipient.

13. A compound of formula (I) as defined in anyone of claims 1 to 10 as well as its tautomers and pharmaceutically acceptable salts, for inhibiting a USP.

14. The compound according to claim 13 for inhibiting USP7.

15. A compound of formula (I) as defined in anyone of claims 1 to 10 as well as its tautomers and pharmaceutically acceptable salts for treating and/or preventing cancer and metastasis, neurodegenerative diseases, such as Alzheimer's disease and Parkinson's disease, immunological disorders, bone and joint diseases, osteoporosis, arthritis inflammatory disorders, cardiovascular diseases, viral infections and diseases, and/or viral infectivity and/or latency, bacterial infections and diseases.

16. A compound according to claim 15, wherein said viral infections and diseases are chosen from herpes simplex-1 or -2 viral infections, hepatitis A, hepatitis C, SARS coronavirus infection and disease, Epstein-Barr virus, rhinoviral infections and diseases, adenoviral infections and diseases, poliomyelitis.

17. A compound according to claim 15 or 16, wherein said compound inhibits one or more viral cysteine proteases related to USP.

18. A compound according to claim 15 wherein said compound inhibits one or more bacterial cysteine proteases.

19. A compound according to claim 15 or 18, wherein said compound inhibits one or more bacterial cysteine proteases chosen from streptopain, clostripain, staphylococcal cysteine protease, gingipain.

20. A combination comprising a compound according to anyone of claims 1 to 10 with one or more active agents chosen from anti-cancer agents, neurological agents, thrombolytic agents, antioxidant agents. anti-infective, antihypertensive agents, diuretic agents, thrombolytic agents, immunosuppressive agents, cardiovascular agents, immunomodulatory agents, anti-inflammatory agents, antiviral agents, anti-bacterial agents.
